# EUROPEAN PATENT SPECIFICATION

(11) **EP 1 305 410 B1**
(45) Date of publication and mention of the grant of the patent: **12.10.2005**
(21) Application number: 01953264.7
(22) Date of filing: 02.08.2001
(51) Int. Cl.: C12N 15/07

(54) **INSULIN PRODUCING CELL-LINE OBTAINED BY ELECTROFUSION**
DURCH ELEKTROFUSION HERGESTELLTE INSULIN-PRODUZIERENDE ZELLINIE
LIGNEE CELLULAIRE OBTENUE PAR ELECTROFUSION ET PRODUISANT DE L'INSULINE

(30) Priority: 02.08.2000 GB 0018808; 19.04.2001 GB 0109633
(43) Date of publication of application: 02.05.2003
(73) Proprietor: Uutech Limited, Coleraine, County Londonderry BT52 1ST (GB)
(72) Inventor: FLATT, Peter Raymond, Co. Antrim BT56 8NR (GB); MCCLENAGHAN, Neville Hugo, Co. Londonderry BT52 1TZ (GB)
(74) Representative: McNally, Roisin
(86) International application number: PCT/GB2001/003476
(87) International publication number: WO 2002/010346

(56) References cited:
- EP-A- 0 251 107
- MCCLENAGHAN NEVILLE H ET AL: "Characterization of a novel glucose-responsive insulin-secreting cell line, BRIN-BD11, produced by electrofusion." DIABETES, vol. 45, no. 8, 1996, pages 1132-1140, XP001056255 ISSN: 0012-1797
- MCCLENAGHAN NEVILLE H ET AL: "Engineering cultured insulin-secreting pancreatic B-cell lines." JOURNAL OF MOLECULAR MEDICINE (BERLIN), vol. 77, no. 1, January 1999 (1999-01), pages 235-243, XP002190163 ISSN: 0946-2716
- WELSH N: "Gene therapy in diabetes mellitus: promises and pitfalls" CURR OPIN MOL THER, vol. 1, no. 4, August 1999 (1999-08), pages 464-470, XP001056458

## Description

The present invention relates to the production of cell lines which secrete insulin. More specifically the invention relates to insulin producing cell lines derived from human pancreatic islet cells.

The limited supply of human islets and labour-intensive methods for their isolation together with marked functional variability are inherent problems, which greatly restrict the number of biochemical and islet transplantation studies being performed. Generation of human pancreatic B-cell lines would provide, for a first time, a practically unlimited supply of pure insulin-secreting cells which can be grown and harvested with a minimum of effort. Such human cell lines would be extremely useful in studies of pancreatic B-cell biology. Further, such human cell lines would be attractive alternatives to the use of primary tissue in cell transplantation therapies for type 1 diabetes.

A large number of rodent insulinoma cell lines have been developed. Most have been derived from transgenic animals expressing the SV-40 antigen oncogene from the insulinoma gene promoter (Efrat *et al*, 1988; Hanahan, 1985; Santere *et al*, 1981). Most of the cell lines produced by these methods show inherent defects including instability and failure to respond to physiological stimulators of insulin secretion (Efrat *et al*., 1996). However, recent studies have succeeded in duplicating many of the properties of fully differentiated pancreatic B-cells (Knaack *et al*., 1994). For example, a murine cell line from a transgenic mouse in which an insulin promoter SV-40 antigen was inserted under the control of the tetracycline-inducible system, was shown to reverse hyperglycemia in a mouse diabetes model (Efrat *et al*., 1995). These experiments clearly demonstrated the potential of pancreatic B-cell lines for cell transplantation and gene therapy of diabetes (Efrat, 1998).

Human insulin-producing cell lines would be a much more desirable model, as opposed to rodent cell lines, for a number of reasons. For clinical transplantation, a major advantage of human cells over those from other species is that there would be no need to overcome the xenotransplantation barrier (Cooper *et al*., 1994). Allografts are much easier to protect from immune rejection compared with xenografts. Hyperacute rejection of xenografts is caused by recognition of cell surface carbohydrate antigens by naturally occuring antibodies (Cooper *et al*., 1994). Xenografts have raised also numerous ethical and safety concerns, including the introduction of unknown animal pathogens into the human population. Another concern is that immunogenic peptides shed by xenografts may elicit immune responses, which could result in cross-reactivity against host proteins, thus potentially triggering new autoimmune responses (Efrat, 1998). Another advantage of human pancreatic B-cells is that they are likely to be more compatible with the human physiological environment compared with most animal B-cells. Moreover, human endocrine cells differ from other species in some of their biological properties. For example, hepatocyte growth factor (HGF) has been shown to be a potent mitogen for human pancreatic B-cell, while in the mouse it is relatively inactive (Hayek *et al*., 1995; Otonkoski *et al*., 1994).

Despite the overwhelming impetus to produce human insulin-secreting cell lines, it has proven to be extremely difficult to establish such cell lines from the human pancreatic endocrine lineage, compared with the rodent (Efrat *et al*., 1996; Wang *et al*., 1997). One human insulinoma cell line has been developed from a spontaneous insulinoma, but it apparently grows slowly and has lost many differentiated characteristics (Gueli *et al*., 1987). Other investigators have attempted to immortalise foetal or adult human pancreatic B-cells using transfection with oncogenes (Wang *et al*., 1997). However this approach has been foiled by inability of such cells to continue to express insulin and their restricted growth rates *in vitro* (Wang *et al*., 1997).

It is an aim of the present invention to provide a human pancreatic cell line which is capable of secreting insulin.

According to the present invention there is provided a human pancreatic cell line, produced by electrofusion of normal human islet cells with immortal human cell lines wherein the human pancreatic cell-line is capable of secreting insulin.

Typical cell lines capable of secreting insulin are chosen from the group of cell lines consisting of the cell-line deposited under Accession No 00112811 at the European Collection of Cell Cultures (ECACC), CAMR, Salisbury, Wiltshire on 28 November 2000 and the cell-lines deposited under Accession Nos PTA 3523, PTA 3524 and PTA 3525 at the American Type Culture Collection, 10801 University Boulevard, Manassas, Virginia 20100-2209, USA on 17 July 2001.

The invention also provides a process for the production of human pancreatic cell lines capable of secreting insulin, the process including the steps of electrofusing a mixture of normal human islet cells with cells from at least one immortal human cell line and incubating the mixture to generate hybrid cells which are capable of secreting insulin.

Preferably the human islet cells and immortal human cells are mixed in an approximate 1:1 ratio.

Preferably electrofusion occurs in a helical chamber.

Preferably the electrofusion step includes exposing the cells to a first pulse phase of AC field, a second pulse phase of DC field and a third pulse phase of AC field.

The first pulse phase may be comprised of between 5 to 10V, preferably 7V, 2MHZ of AC field for between 20-40, preferably 30 seconds.

The second pulse phase typically comprises of 40-80V, preferably 60V, triple pulses of DC field with each of the triple pulses being 10-20, preferably 15 seconds in duration.

The third pulse phase typically comprises of 5-10V, preferably 7V, 2MHZ of AC field for 20-40, preferably 30 seconds.

The media in which the cells are incubated typically comprises hypoxanthine, aminopterin and thymidine.

Typically, hypoxanthine is present in the incubating media at a concentration of between 0.05µmol/l to 0.5µmol/l.

Typically, aminopterin is present in the incubating media at a concentration of between 0.2-0.6 µmol/l.

Typically, thymidine is present in the incubating media at a concentration of between 10-20 µmol/l.

The incubation may be carried out in the presence of at least one secretagogue chosen from the group comprising glucose, glyceraldehyde, arginine, leucine and alanine.

The incubation may be carried out in the presence of at least one substance chosen from the group comprising KCl, IBMX, thioglucose, tolbutamide, diazoxide and verapamil.

A cell line produced by the process of the invention may exhibit glucose transport characteristics as efficient as normal pancreatic B cells.

A cell line produced by the process of the invention may exhibit glucose phosphorylating activity consistent with normal pancreatic cells.

The invention also relates to the use of insulin producing cells produced by the process of the invention to provide gene therapy for type 1 diabetes.

The invention also relates to the use of insulin producing cells produced by the process in the preparation of a medicament for the treatment of diabetes.

The invention also relates to the use of a cell-line according to the invention in the preparation of a medicament for the treatment of diabetes.

The invention also relates to the use of a cell-line according to the invention for the production of insulin.

The invention also relates to cells or materials derived from the cell-lines disclosed herein and to uses of these.

According to the present invention there is also provided a process for the generation of human pancreatic cell lines which are capable of secreting insulin, the process including the step of electrofusing normal human islet cells with immortal human cell lines to generate hybrid cells which are capable of expressing attributes of normal pancreatic B-cells.

The invention thus provides human pancreatic cell-lines capable of secreting insulin produced by an electrofusing process.

In the examples the cell-line referred to under Accession numbers above are referred under internal reference numbers. These are as follows:

| Accession No | Internal Reference |
|---|---|
| 00112811 | 1.1B4 |
| PTA 3523 | 1.1E7 |
| PTA 3524 | 1.2B4 |
| PTA 3525 | 1.4E7 |

A number of embodiments of the present invention will now be described by way of example only. With reference to the accompanying figures in which:

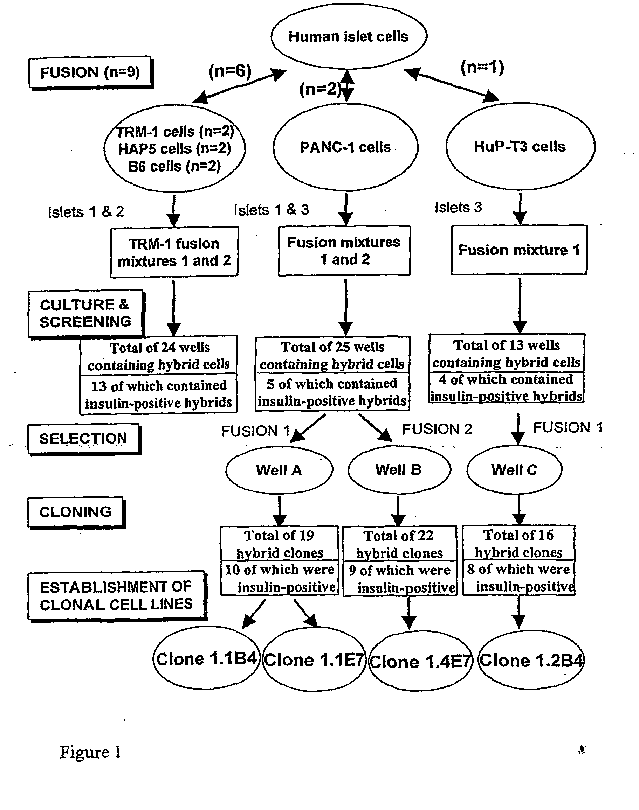

Figure 1 shows the typical steps involved in the production of human insulin-secreting pancreatic B-cell lines by electrofusion.

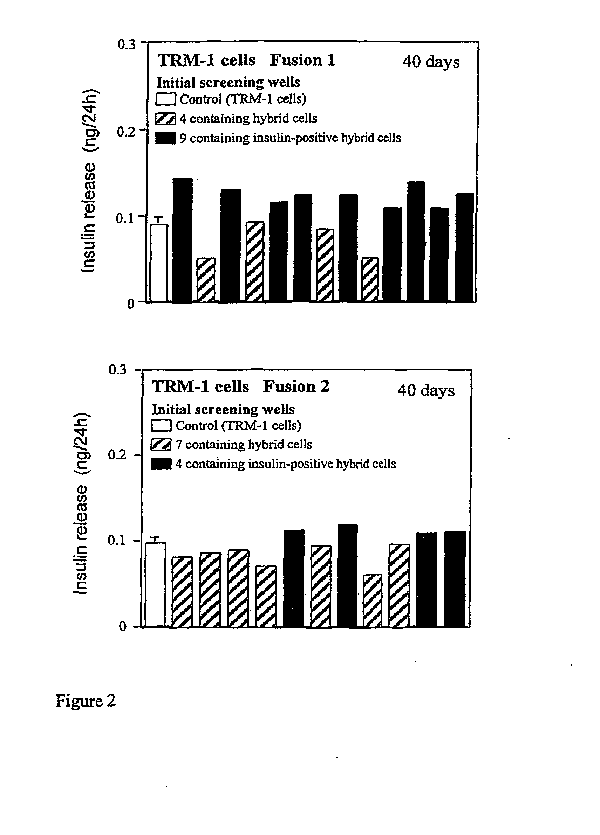

Figure 2 represents the insulin released into the medium in the final 24 hours by cell hybrids (azaguanine resistant) resulting from electrofusion of human islet cells with TRM-1 cells after 40 days culture, control values are mean ± sem (n=6), other values are mean of duplicate determinations, fusions 1 and 2 utilised islet preparations 1 and 2, respectively.

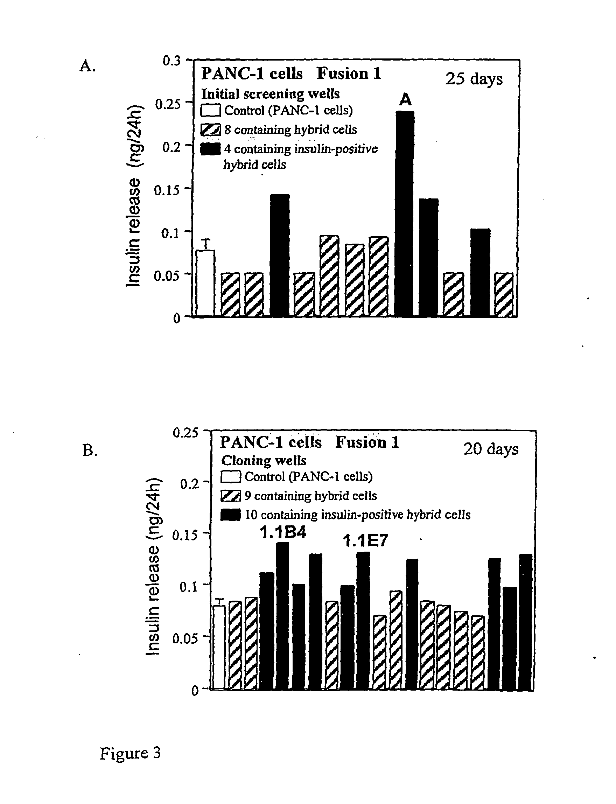

Figure 3 represents the insulin released into the medium in the final 24 hours by cell hybrids (azaguanine resistant) resulting from electrofusion of human islet cells with PANC-1 cells after 25 days culture, cells in A were selected on the basis on high insulin output for cloning, cells in B, clones 1.1B4 and 1.1E7 were selected on the basis of insulin output for further evaluation, control values are mean ± sem (n=6), other values are mean of duplicate determinations, fusion utilised islet preparation 1.

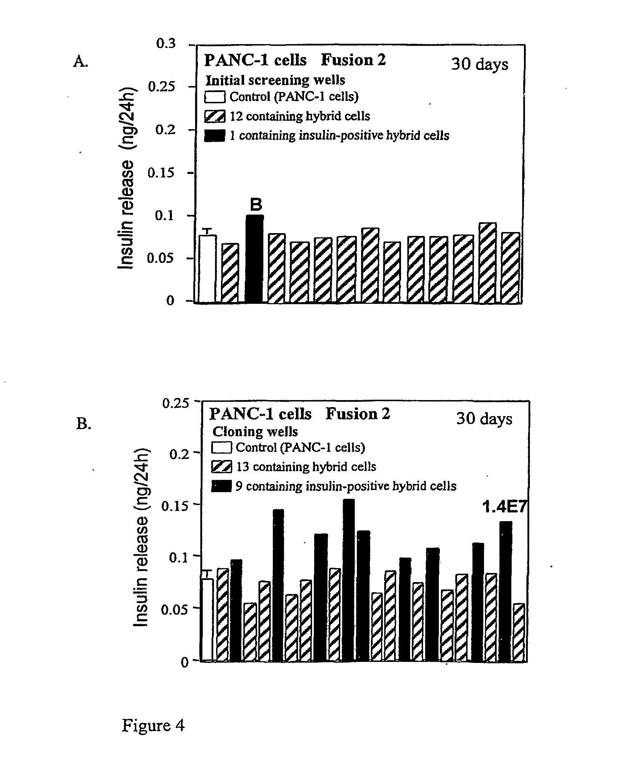

Figure 4 represents the insulin released into the medium in the final 24 hours by cell hybrids (azaguanine resistant) resulting from electrofusion of human islet cells with PANC-1 cells after 30 days culture, cells in well B were selected on the basis on high insulin output for cloning, (B)clone 1.1E7 was selected for further evaluation, control values are mean ± sem (n=6), other values are mean of duplicate determinations, fusions utilised islet preparation 3.

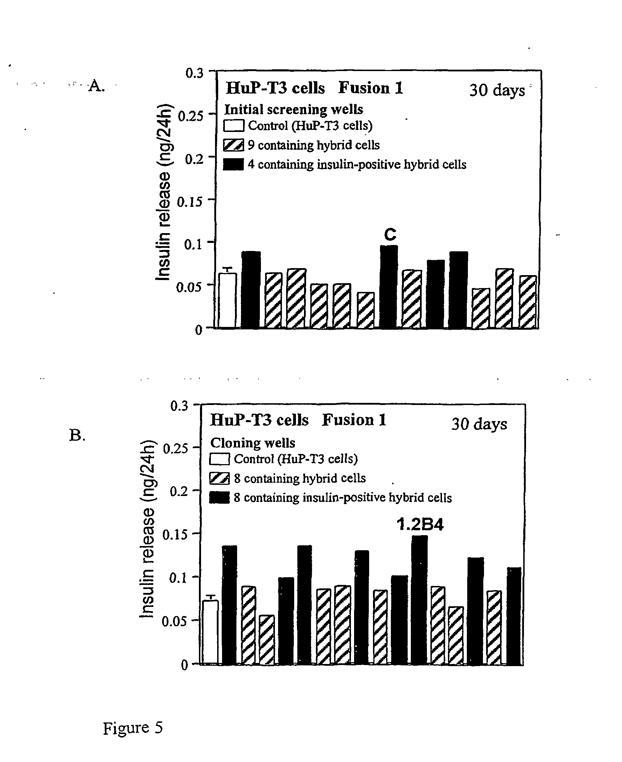

Figure 5 represents the insulin released into the medium in the final 24 hours by cell hybrids (azaguanine resistant) resulting from electrofusion of human islet cells with Hup-T₃-1 cells after 30 days culture, cells in well C were selected on the basis on high insulin output for cloning, (B) clones 1.2B4 was selected for further evaluation, control values are mean ± sem (n=6), other values are mean of duplicate determinations, fusion utilised islet preparation 3.

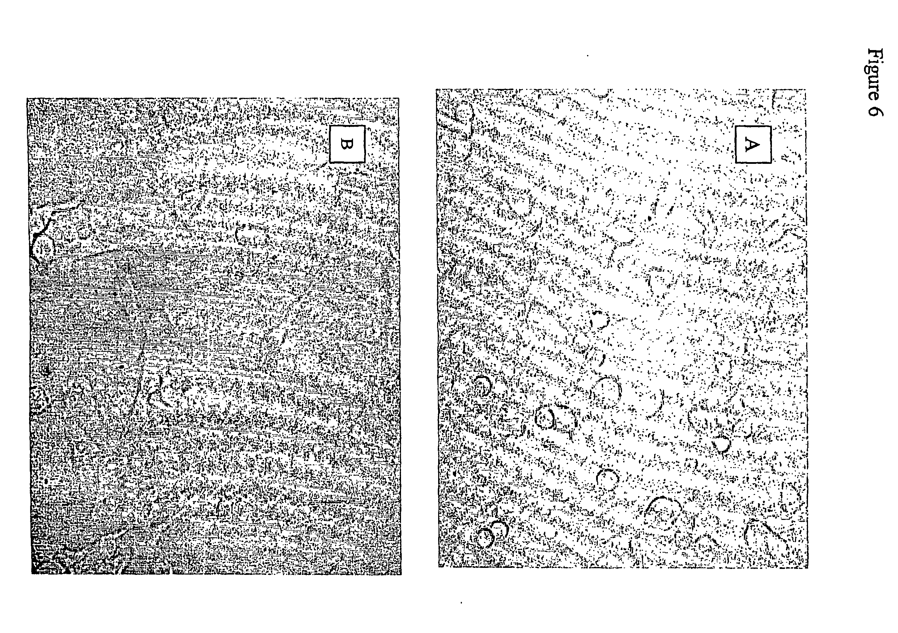

Figure 6 shows, (A) the mophology of PANC-1 cell lines and (B) the morphology of human islet-derived 1.1B4 cell lines, using phase contrast microscopy (x200 magnification).

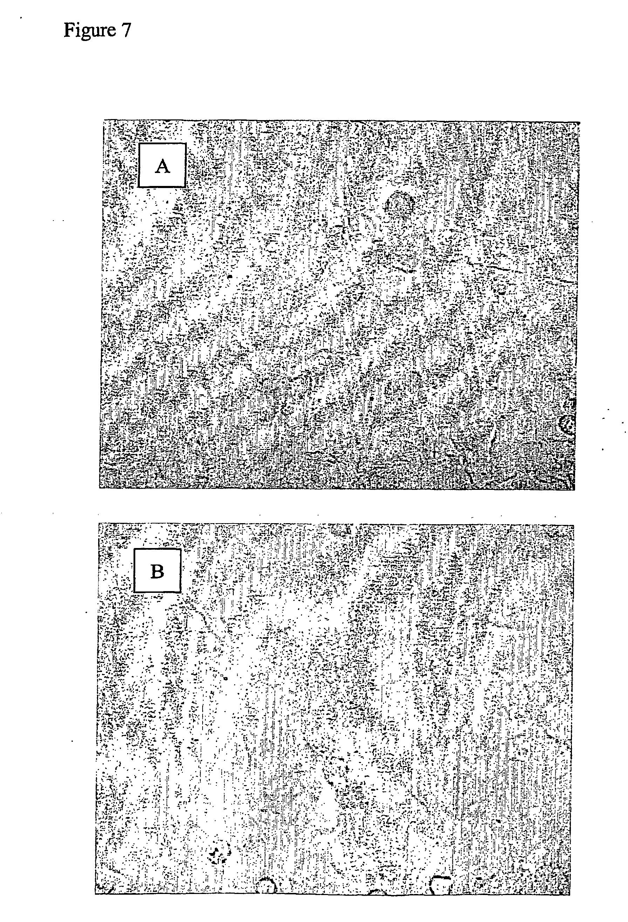

Figure 7 shows (A) the morphology of human-islet derived 1.1E7 cell line and (B) 1.4E7 cell line, using phase contrast microscopy (x200 magnification).

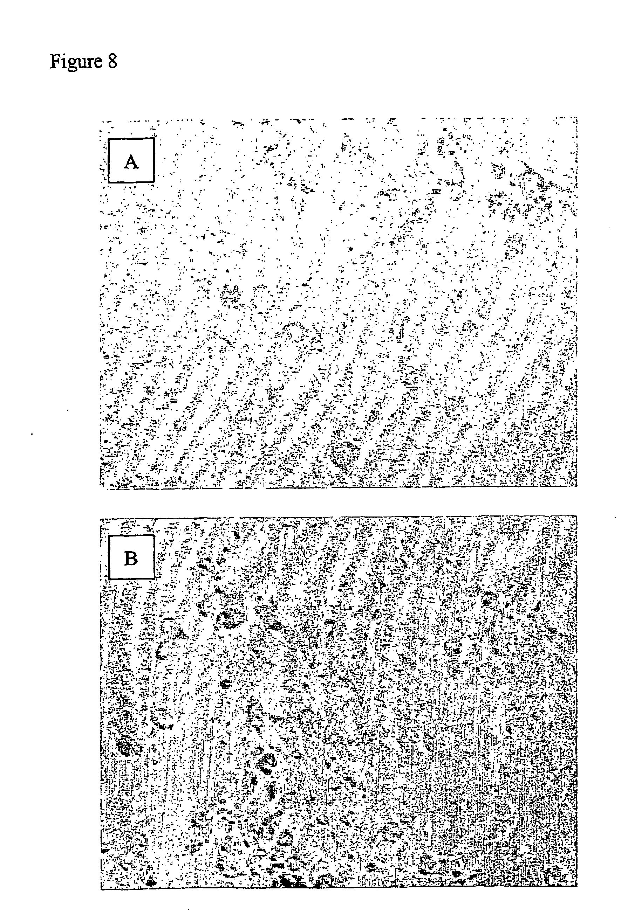

Figure 8 shows (A) the morphology of Hup-T₃ cell line and (B) 1.2B4 human islet-derived cell line, using phase contrast microscopy (x200 magnification).

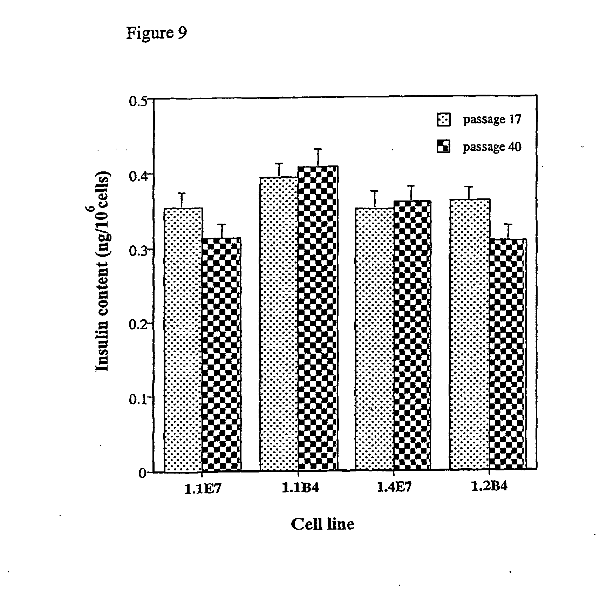

Figure 9 represents the cellular insulin content of human islet-derived cell lines at different passages, values are mean ± sem (n=8), there were no significant differences in insulin content between cell lines or increasing passage number.

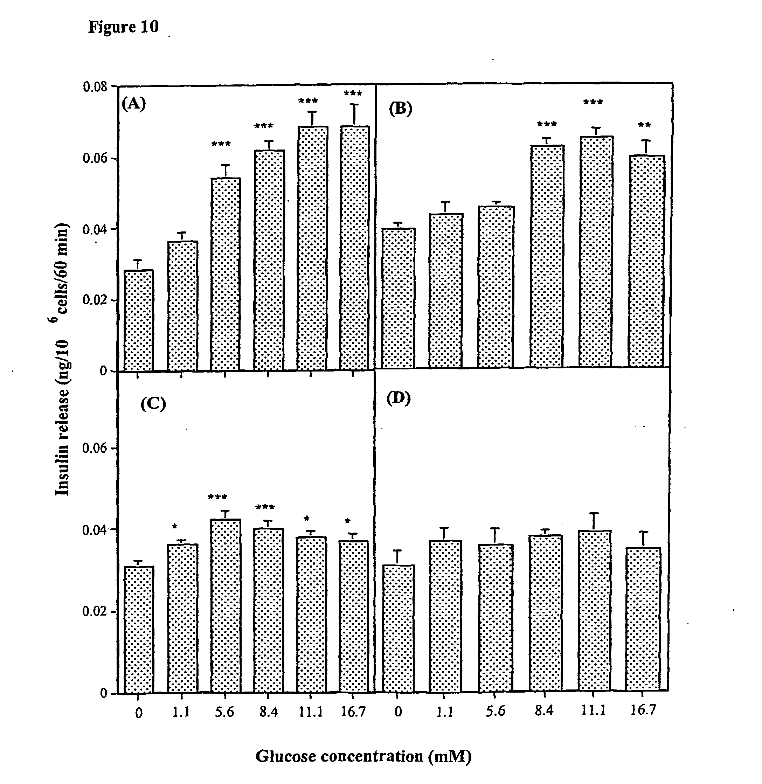

Figure 10 represents the glucose responsiveness of human islet-derived 1.1B4 (A), 1.1E7 (B), 1.4E7 (C) and 1.2B4 (D) cells, following 40 min of preincubation, effects of various glucose concentrations were tested during 60 min incubations, values are mean ± sem (n=12), *ρ <0.05, **ρ<0.01, ***ρ<0.001 compared with 0 mM glucose.

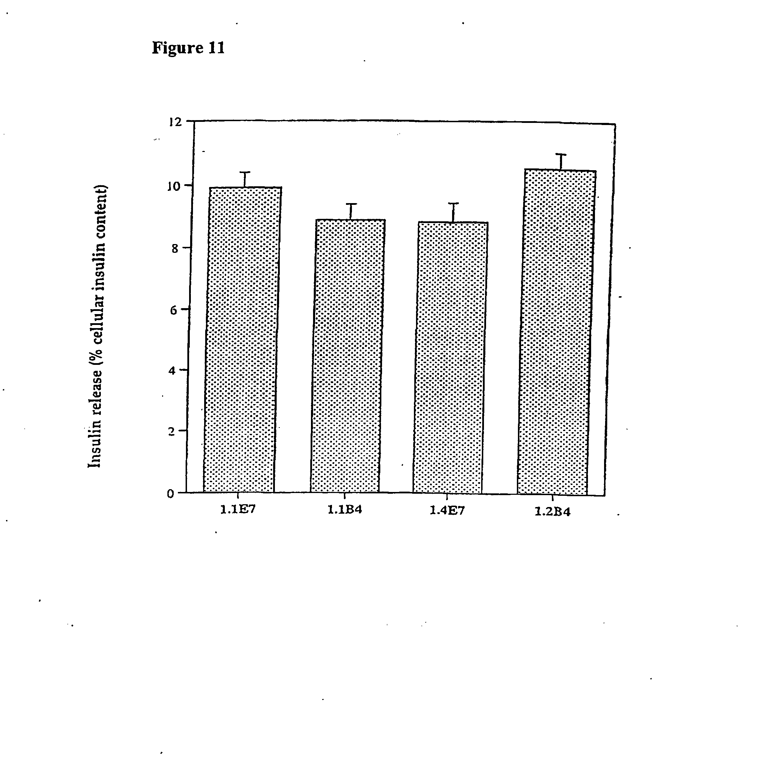

Figure 11 represents the insulin released from human islet-derived clones in 1.1 mM glucose as a % of the cellular insulin content, following 40 min preincubation, effects of 1.1 mM glucose were tested during 60 min incubation, values are mean ± sem (n=12).

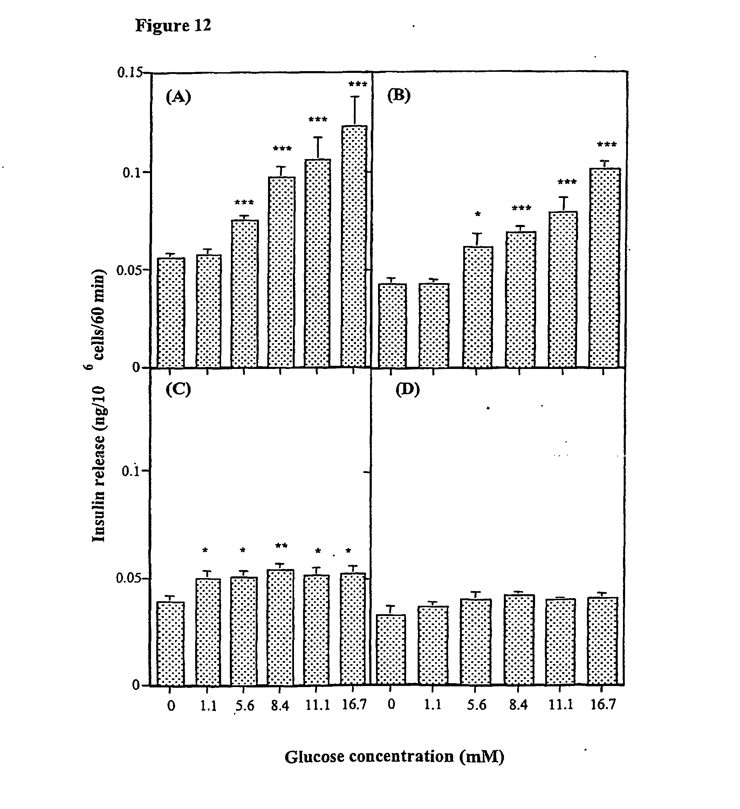

Figure 12 represents the glucose responsiveness of human islet-derived 1.1B4 (A), 1.1E7 (B) 1.4E7 (C) and 1.2B4 (D) cells in the presence of IBMX (200 µM), following 40 min preincubation, effects of various glucose were tested in the presence of IBMX (200 µM) during 60 min incubations, values are mean ± sem (n=12), *ρ <0.05, **ρ<0.01, ***ρ<0.001 compared with 0 mM glucose in presence of 200 µM IBMX).

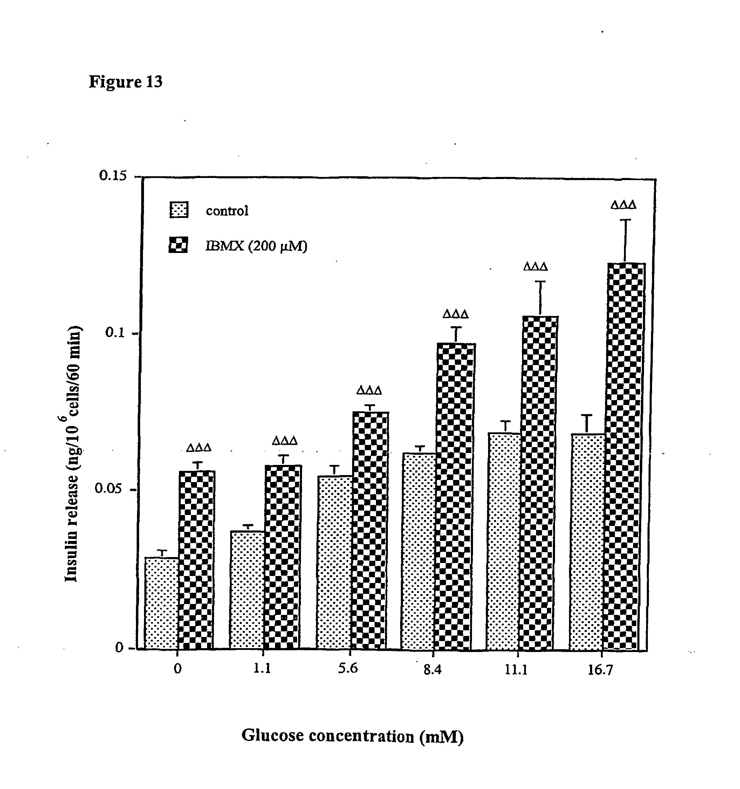

Figure 13 represents the effects of IBMX (200 µM) on glucose responsiveness of human islet-derived 1.1B4 cells, following 40 min preincubation, insulin secretion was measured after 60 min incubations in the presence of the indicated secretagogues, values are mean ± sem (n=12), ΔΔΔρ <0.001 compared with control at the same glucose concentration.

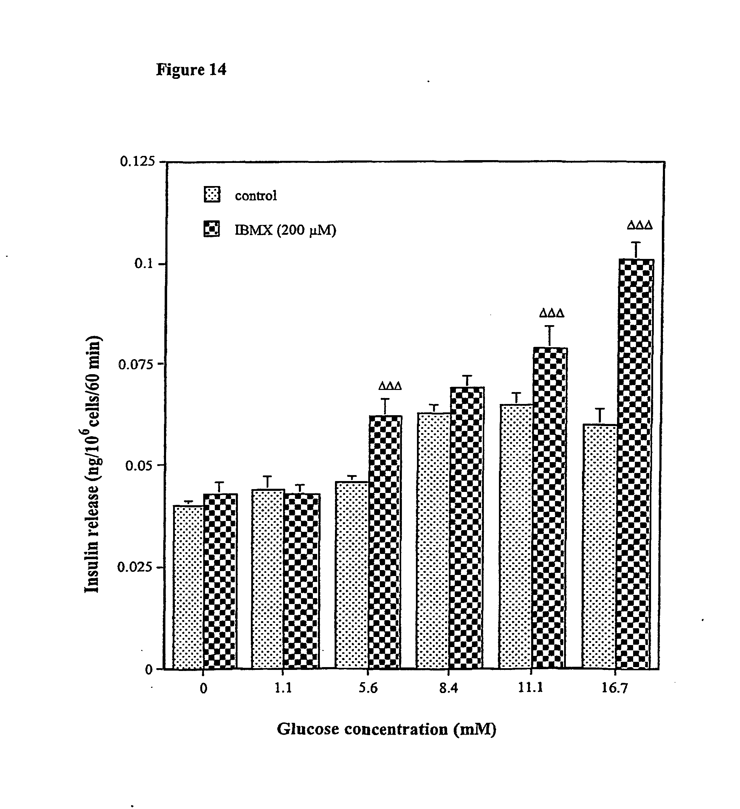

Figure 14 represents the effects of IBMX (200 µM) on glucose responsiveness of human islet-derived 1.1E7 cells, following 40 min preincubation, insulin secretion was measured after 60 min incubations in the presence of the indicated secretagogues, values are mean ± sem (n=12), ΔΔΔρ <0.001 compared with control at the same glucose concentration.

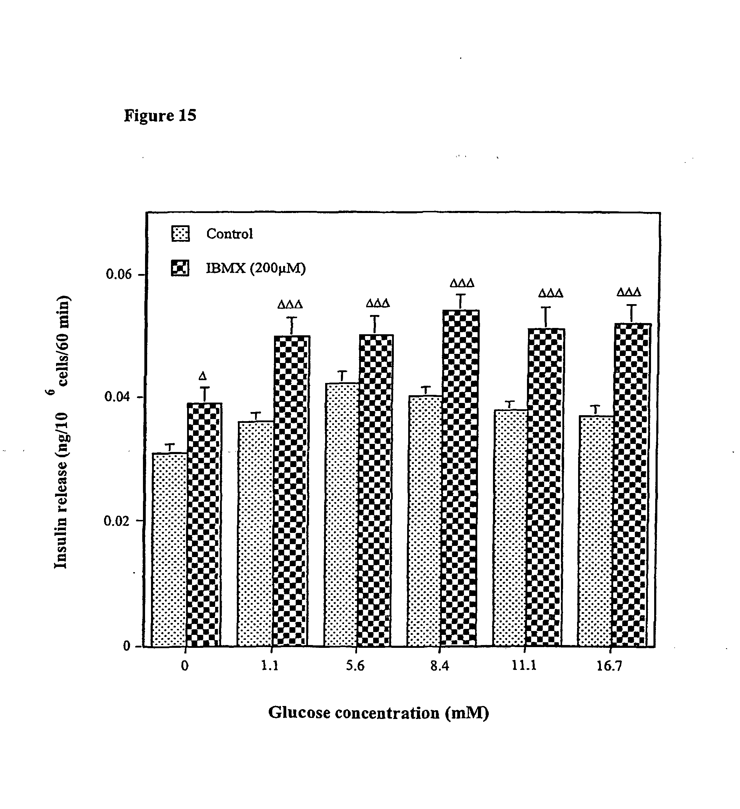

Figure 15 represents the effects of IBMX (200 µM) on glucose responsiveness of human islet-derived 1.4E7 cells, following 40 min preincubation, insulin secretion was measured after 60 min incubations in the presence of the indicated secretagogues, values are mean ± sem (n=12), Δρ <0.05, ΔΔΔρ <0.001 compared with control at the same glucose concentration.

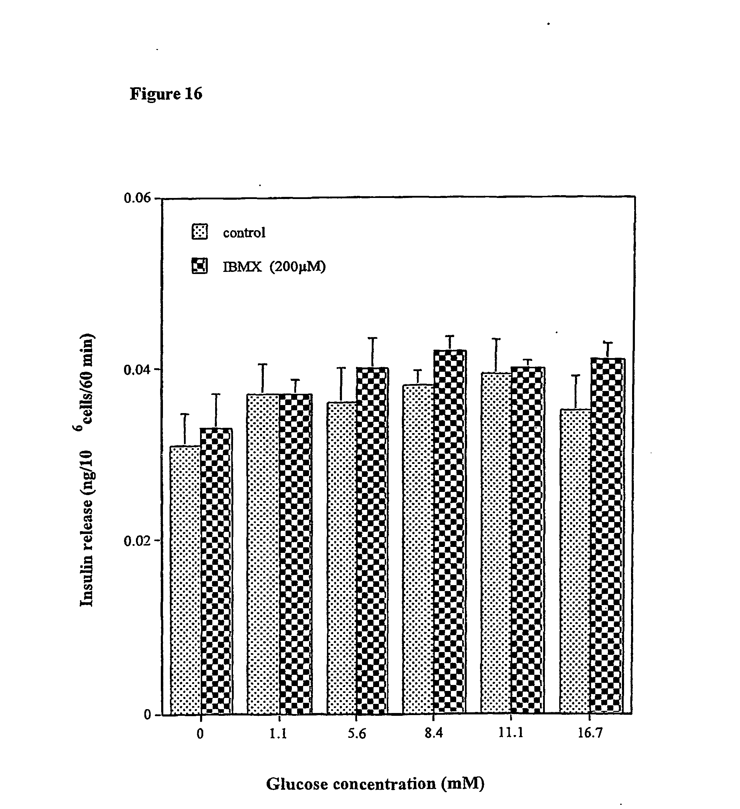

Figure 16 represents the effects of IBMX (200 µM) on glucose responsiveness of human islet-derived 1.2B4 cells, following 40 min of preincubation, insulin secretion was measured after 60 min incubations in the presence of the indicated secretagogues, values are mean ± sem (n=12).

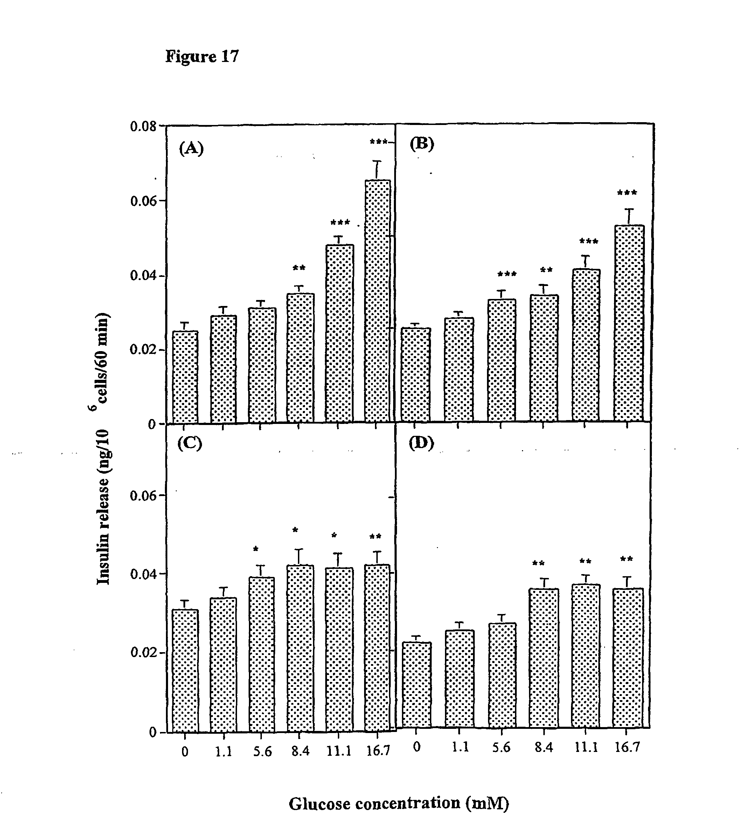

Figure 17 represents the glucose responsiveness of human islet-derived 1.1B4 (A), 1.1E7 (B), 1.4E7 (C) and 1.2B4 cells in the presence of 5-thioglucose (2 mM), following 40 min of preincubation, effects of various glucose concentrations were tested in the presence of 5-thioglucose (2 mM) during 60 min incubations, values are mean ± sem (n=12) *ρ <0.05, **ρ <0.01, ***ρ<0.001.

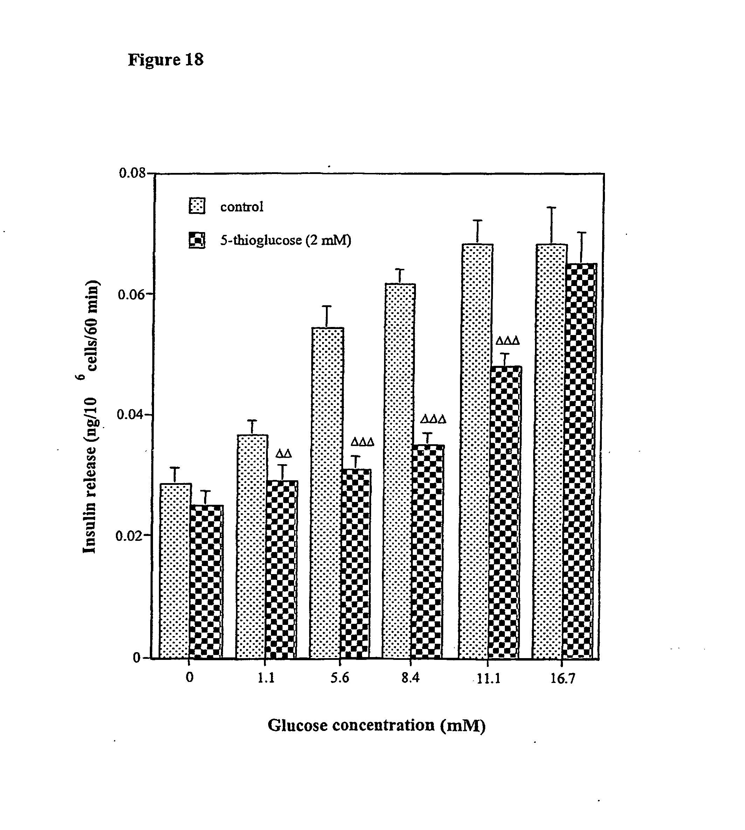

Figure 18 represents the effects of 5-thioglucose (2 mM) on glucose responsiveness of human islet-derived 1.1B4 cells, following 40 min preincubation, insulin secretion was measured after 60 min incubations in the presence of the indicated secretagogues, values are mean ± sem (n=12), ΔΔρ <0.01, ΔΔΔρ <0.001 compared with control at the same glucose concentration.

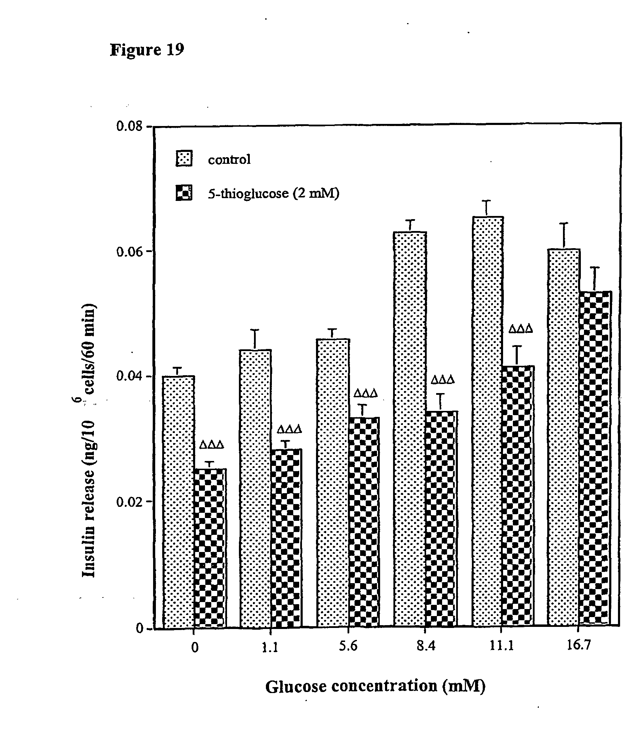

Figure 19 represents the effects of 5-thioglucose (2 mM) on glucose responsiveness of human islet-derived 1.1E7 cells, following 40 min preincubation, insulin secretion was measured after 60 min incubations in the presence of the indicated secretagogues, values are mean ± sem (n=12), ΔΔΔρ <0.001 compared with control at the same glucose concentration.

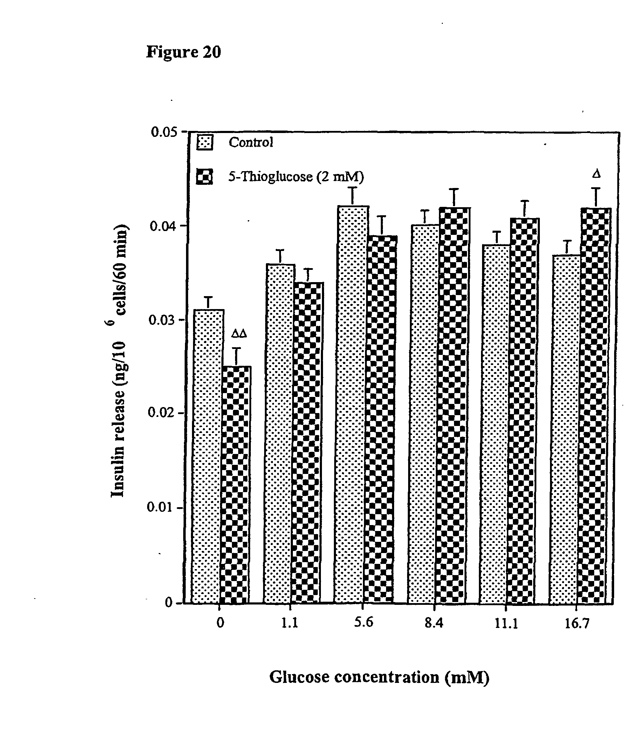

Figure 20 represents the effects of 5-thioglucose (2 mM) on glucose responsiveness of human islet-derived 1.1E7 cells, following 40 min preincubation, insulin secretion was measured after 60 min incubations in the presence of the indicated secretagogues, values are mean ± sem (n=12), Δρ<0.05, ΔΔρ <0.01 compared with control at the same glucose concentration.

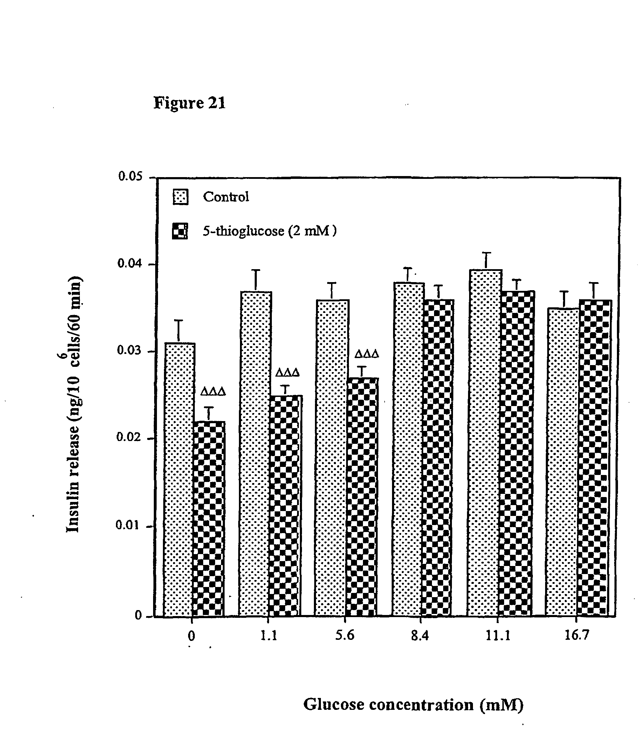

Figure 21 represents the effects of 5-thioglucose (2 mM) on glucose responsiveness of human islet-derived 1.2B4 cells, following 40 min preincubation, insulin secretion was measured after 60 min incubations in the presence of the indicated secretagogues, values are mean ± sem (n=12), ΔΔΔρ <0.001 compared with control at the same glucose concentration.

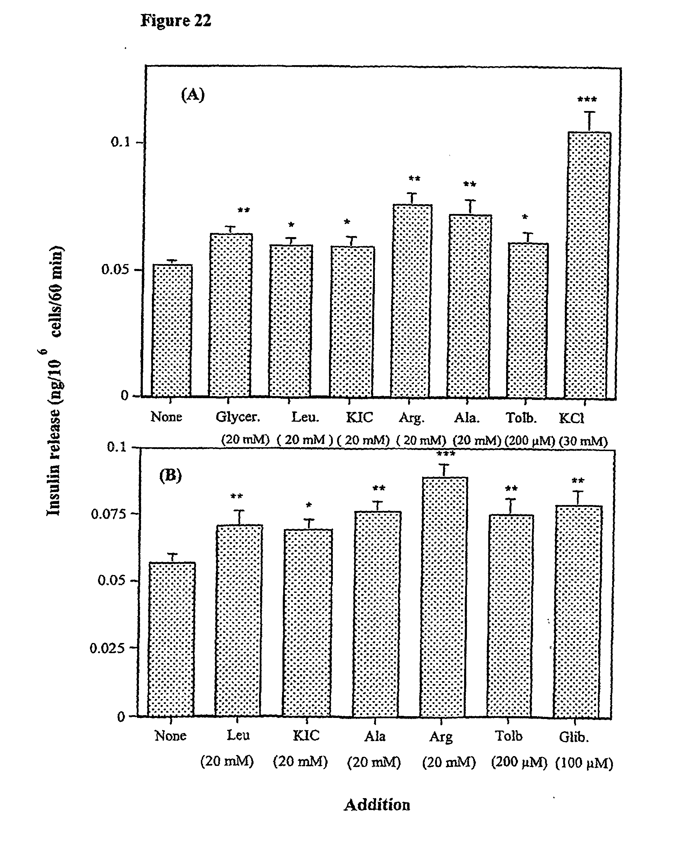

Figure 22 represents the effects of known stimulators of pancreatic B-cell function on insulin secretion from human islet-derived 1.1B4 cells at 5.6 or 11.1 mM glucose, graph A and B respectively, following 40 min of preincubation, effects of various additions at 5.6 (A) or 11.1 (B) mM glucose were tested, values are mean ± sem (n=12), *ρ<0.05, **ρ<0.01, ***ρ <0.001 compared with 5.6 (A) or 11.1 (B) mM glucose alone.

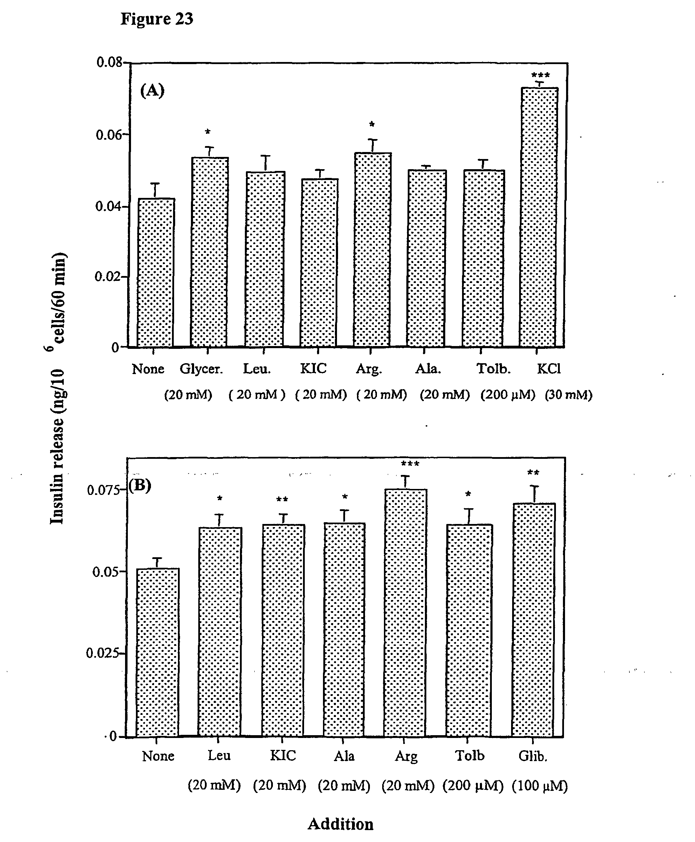

Figure 23 represents the effects of known stimulators of pancreatic B-cell function on insulin secretion from human islet-derived 1.1E7 cells at 5.6 or 11.1 mM glucose, graph A and B respectively, following 40 min of preincubation, effects of various additions at 5.6 (A) or 11.1 (B) mM glucose were tested, values are mean ± sem (n=8), *ρ<0.05, **ρ<0.01, ***ρ <0.001 compared with5.6 (A) or 11.1 (B) mM glucose alone.

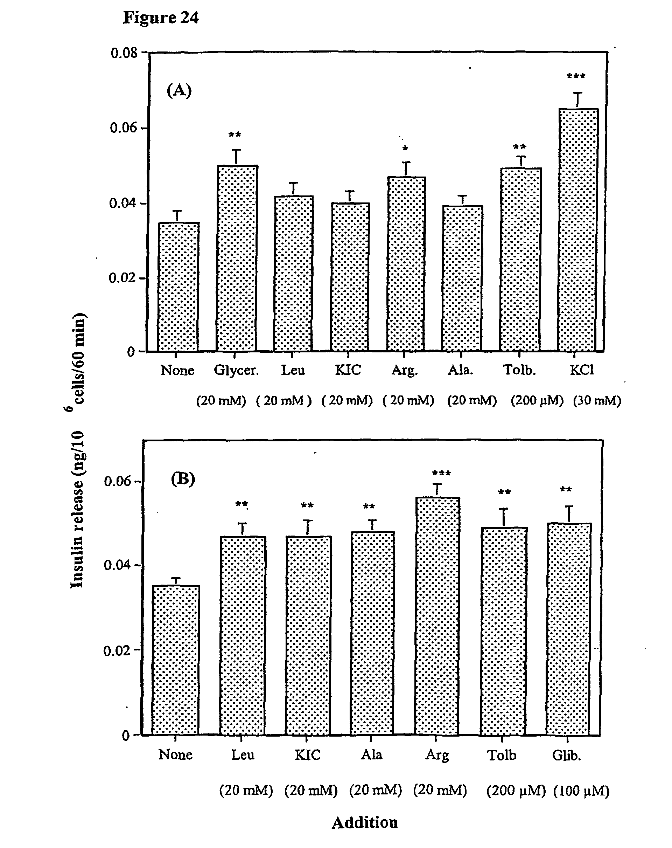

Figure 24 represents the effects of known stimulators of pancreatic B-cell function on insulin secretion from human islet-derived 1.1E7 cells at 5.6 or 11.1 mM glucose, graph A and B respectively, following 40 min of preincubation, effects of various additions at 5.6 (A) or 11.1 (B) mM glucose were tested, values are mean ± sem (n=12), *ρ<0.05, **ρ<0.01, ***ρ <0.001 compared with5.6 (A) or 11.1 (B) mM glucose alone.

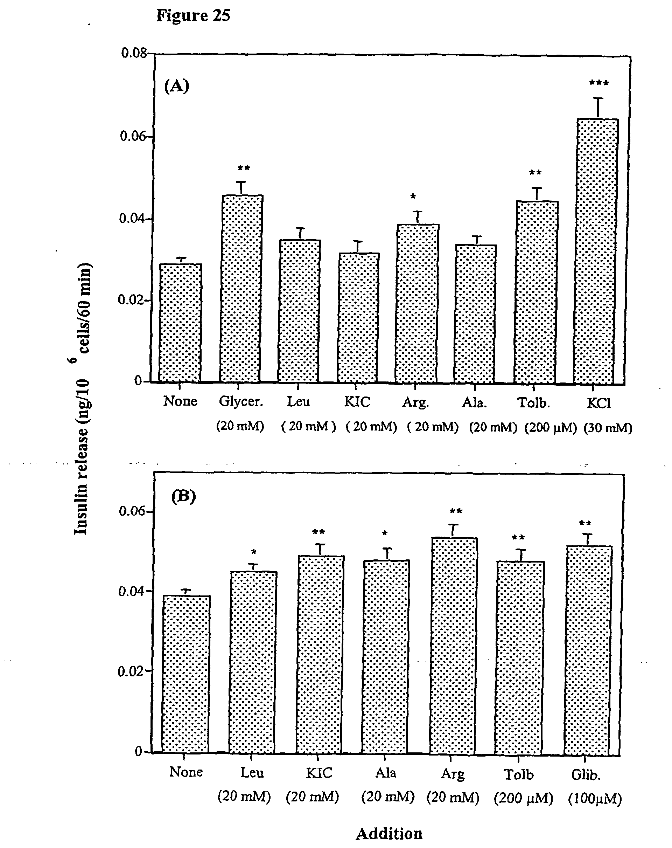

Figure 25 represents the effects of known stimulators of pancreatic B-cell function on insulin secretion from human islet-derived 1.2B4 cells at 5.6 or 11.1 mM glucose, graph A and B respectively, following 40 min of preincubation, effects of various additions at 5.6 (A) or 11.1 (B) mM glucose were tested, values are mean ± sem (n=12), *ρ<0.05, **ρ<0.01, ***ρ <0.001 compared with 5.6 (A) or 11.1 (B) mM glucose alone.

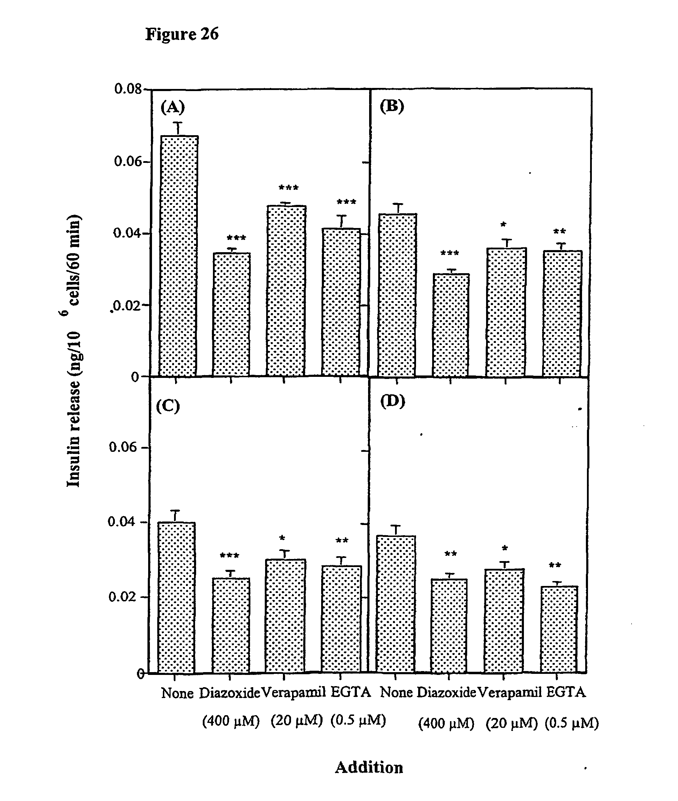

Figure 26 represents the effects of known stimulators of pancreatic B-cell function on insulin secretion from human islet-derived 1.1B4, 1.1E7, 1.4E7 and 1.2B4 cells, graph A, B, C and D respectively, following 40 min of preincubation, effects of various additions at 16.7 mM glucose during 60 min incubations, values are mean ± sem (n=12), *ρ<0.05, **ρ<0.01, ***ρ <0.001 compared with 16.7 mM glucose alone, effects of EGTA were determined in calcium free buffer.

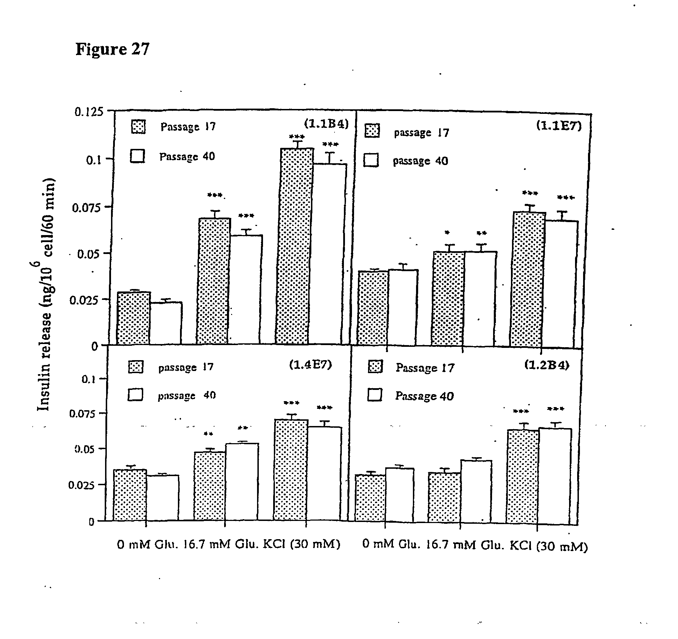

Figure 27 represents the effects of passage number on stimulating insulin secretion from human islet-derived insulin secreting cell lines, following 40 min of preincubation, effects of various additions were tested during a 60 min incubation period, values are mean ± sem (n=12), *ρ<0.05, **ρ<0.01, ***ρ <0.001 compared with 0 mM glucose, there were no significant differences between passage 17 and 40.

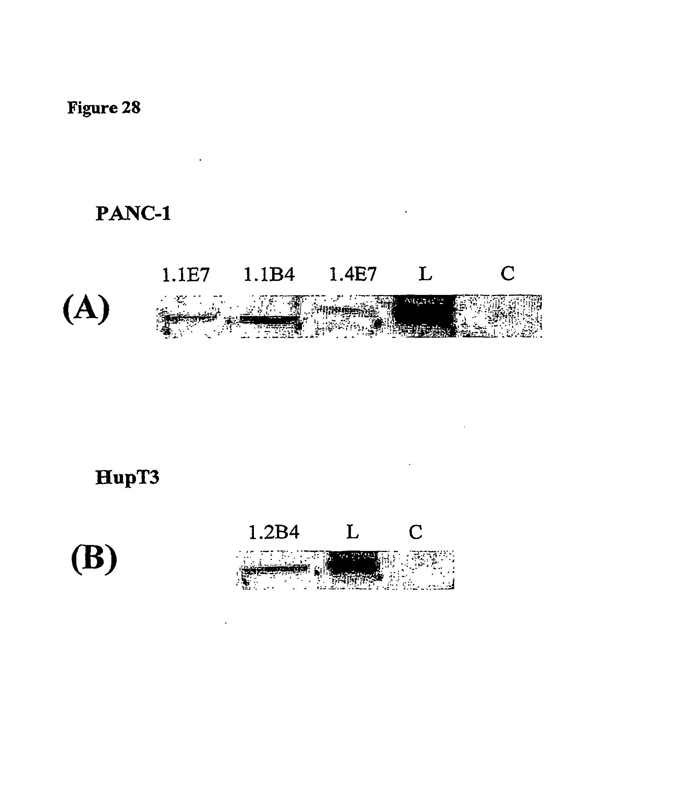

Figure 28 shows a western blotting analysis for GLUT-1 glucose transporter protein in (A) PANC-1 and derived clonal human islet cells and (B) Hup-T₃ and derived clonal human islet cells, where L is liver from Wistar rat which was used as a positive control and C represents parental cells of PANC-1 and Hup-T₃ respectively.

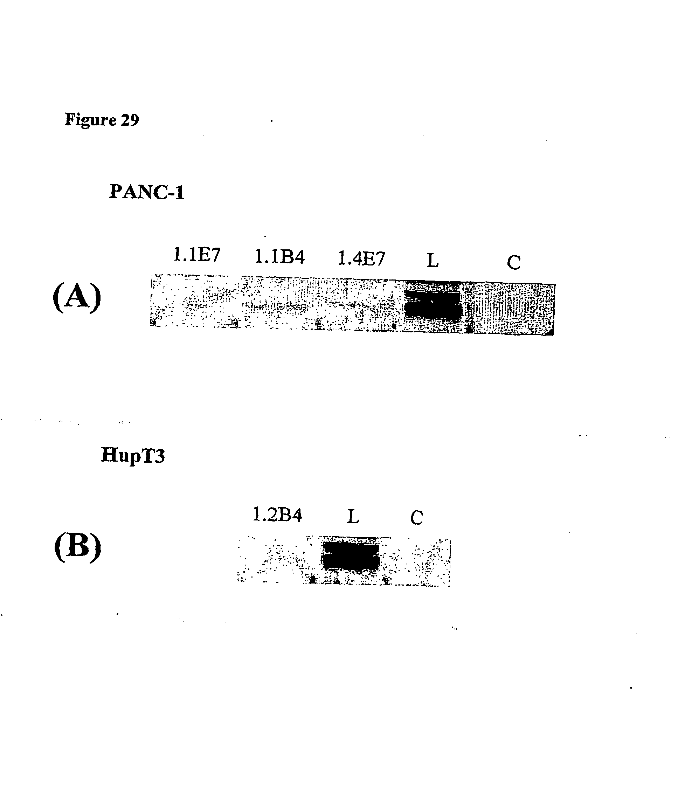

Figure 29 shows a western blotting analysis for glucokinase protein in (A) PANC-1 and derived clonal human islet cells and (B) Hup-T₃ and derived clonal human islet cells, where L is liver from Wistar rat which was used as a positive control and C represents parental cells of PANC-1 and Hup-T₃ respectively.

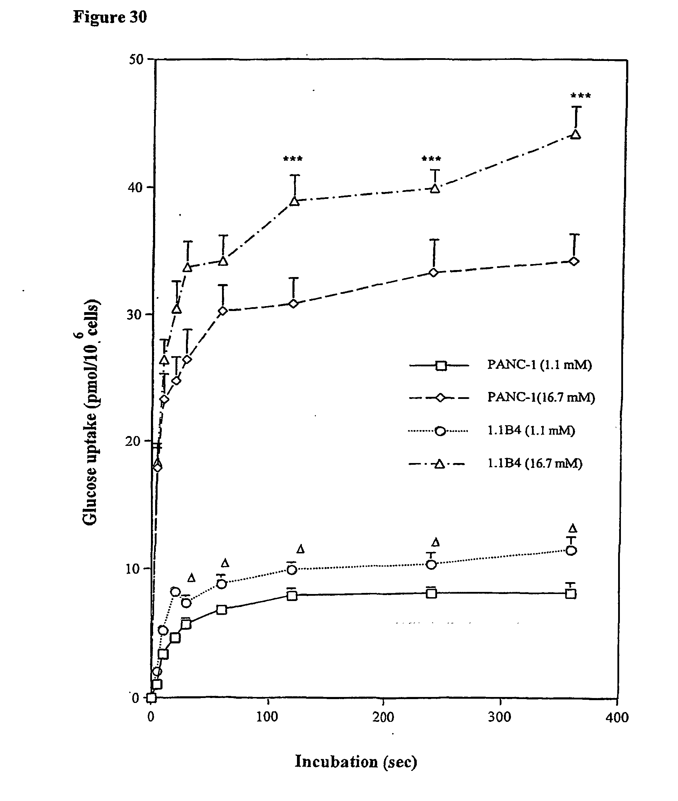

Figure 30 illustrates 3-O-methyl-D-[1-³H]glucose uptake by PANC-1cells or human islet-derived 1.1B4 cells at low and high glucose concentration, incubations were performed at 37°C at 1.1 or 16.7 mM glucose for periods of 0 to 360 seconds. Values are mean ± sem (n=3), ***ρ <0.001 when compared with PANC-1 at 16.7 mM glucose, Δρ <0.05 when compared with PANC-1 at 1.1mM glucose.

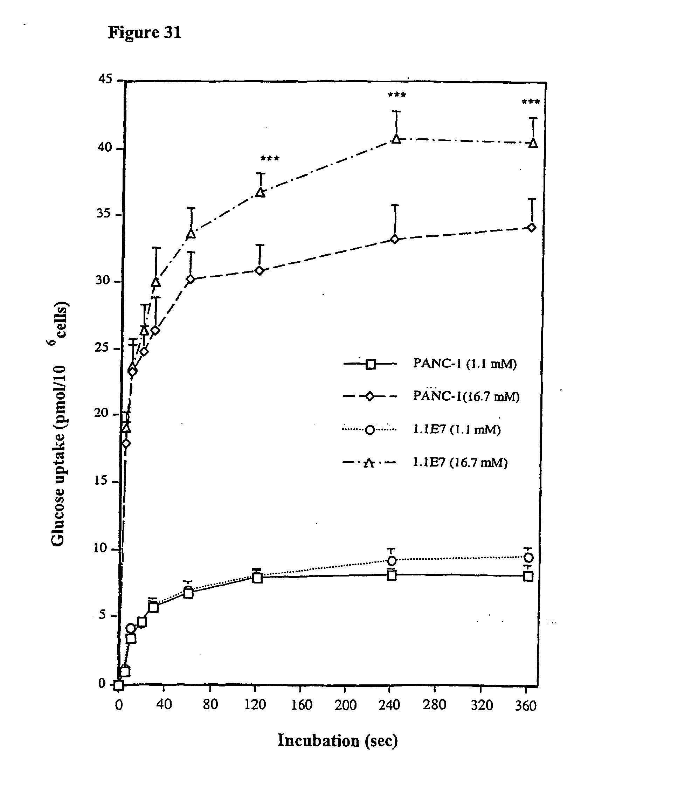

Figure 31 illustrates 3-O-methyl-D-[1-³H]glucose uptake by PANC-1cells or human islet-derived 1.1E7 cells at low and high glucose concentration, incubations were performed at 37°C at 1.1 or 16.7 mM glucose for periods of 0 to 360 seconds, values are mean ± sem (n=3), ***ρ <0.001 when compared with PANC-1 at 16.7 mM glucose.

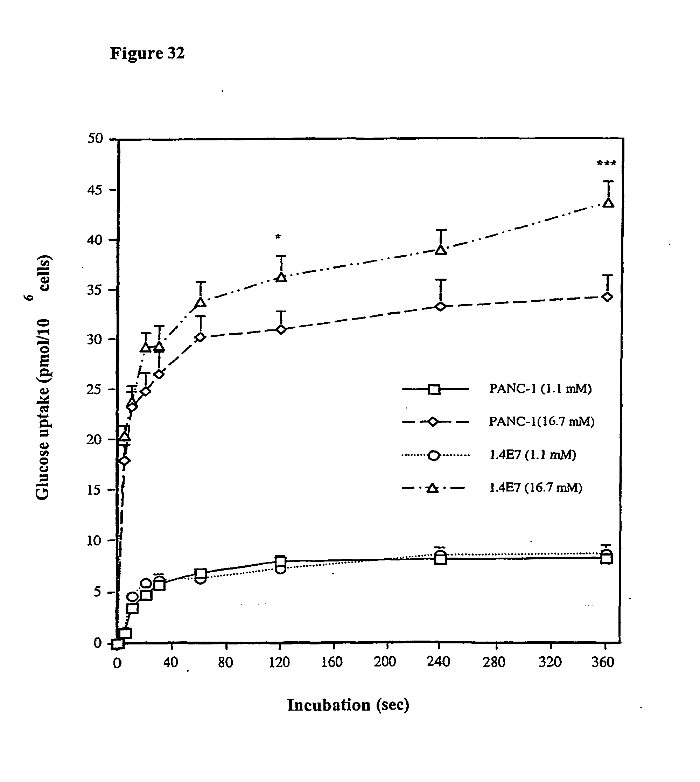

Figure 32 illustrates 3-O-methyl-D-[1-³H]glucose uptake by PANC-1cells or human islet-derived 1.1E7 cells at low and high glucose concentration, incubations were performed at 37°C at 1.1 or 16.7 mM glucose for periods of 0 to 360 seconds, values are mean ± sem (n=3), *ρ <0.05, ***ρ <0.001 when compared with PANC-1 at 16.7 mM glucose.

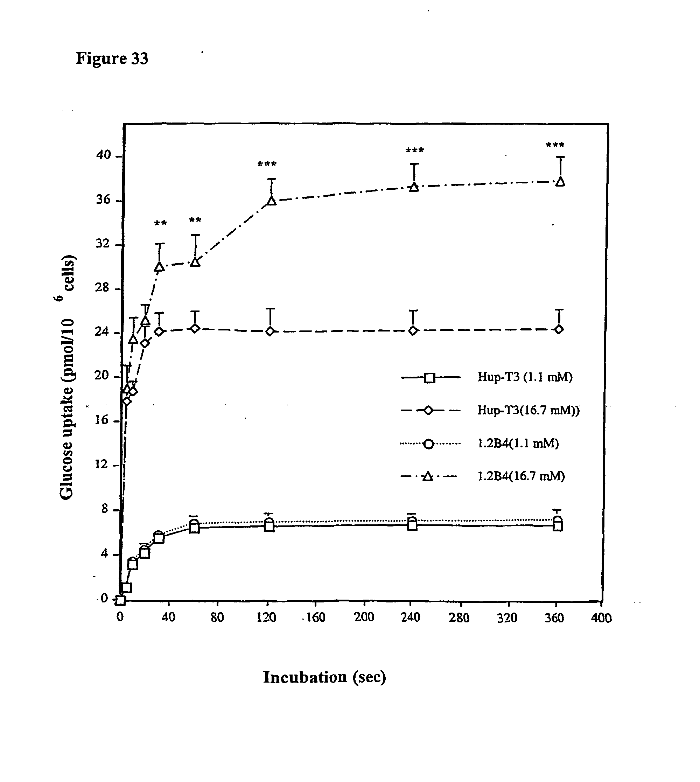

Figure 33 illustrates 3-O-methyl-D-[1-³H]glucose uptake by Hup-T₃ cells or human islet-derived 1.2B4 cells at low and high glucose concentration, incubations were performed at 37°C at 1.1 or 16.7 mM glucose for periods of 0 to 360 seconds, values are mean ± sem (n=3), *ρ <0.01, ***ρ <0.001 when compared with Hup-T₃ at 16.7 mM glucose.

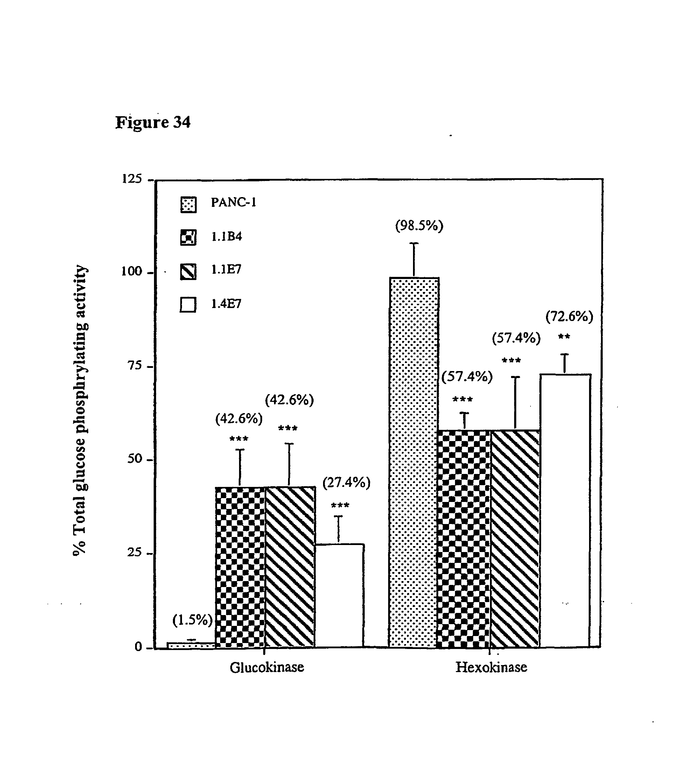

Figure 34 illustrates glucose phosphorylating activities of PANC-1 and human islet-derived 1.1B4, 1.1E7 and 1.4E7 cells, soluble cytoplasmic fractions were assayed spectrophotometrically to determine the relative contributions of glucokinase and hexokinase to the total glucose phosphorylating activities of the cells, values are mean ± sem (n=3), *ρ <0.01, ***ρ <0.001 compared with PANC-1 cells, the percentage contribution to the total glucose phosphorylating activity is given in parentheses.

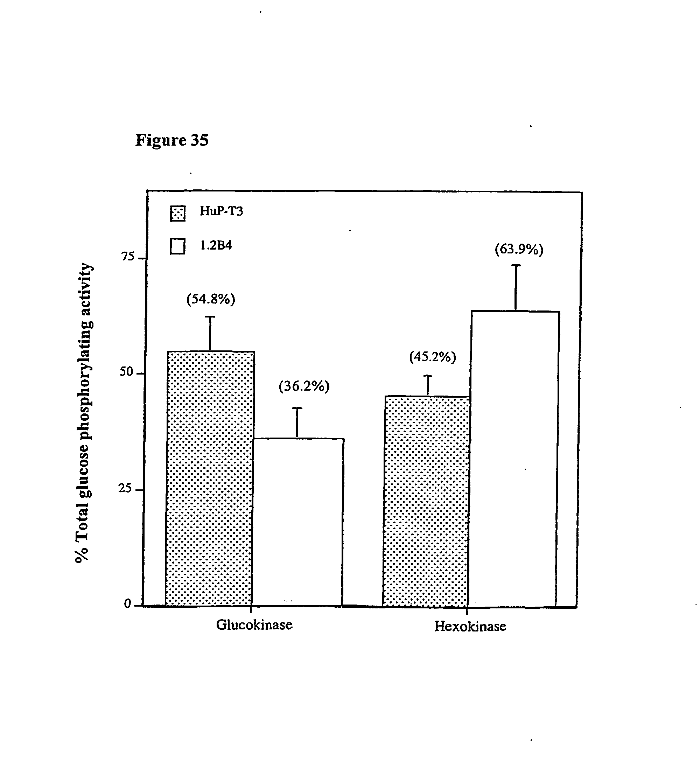

Figure 35 illustrates glucose phosphorylating activities of Hup-T₃ and human islet-derived 1.2B4 cells, soluble cytoplasmic fractions were assayed spectophotometrically to determine the relative contributions of glucokinase and hexokinase to the total glucose phosphorylating activities of the cells, values are mean ± sem (n=3), there were no significant differences between the two cell types, the percentage contribution to the total glucose phosphorylating activity is given in parentheses.

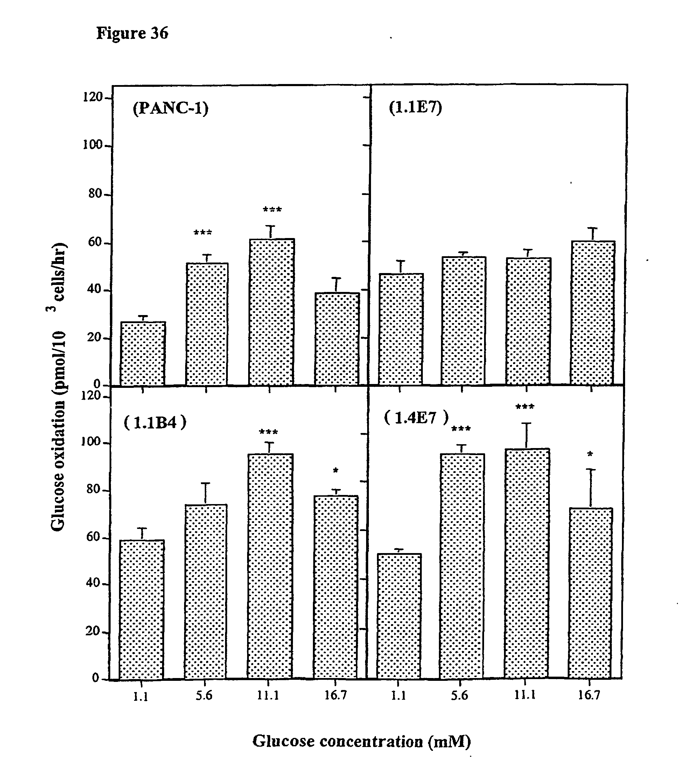

Figure 36 illustrates glucose oxidation from D-[U-¹⁴C]glucose in PANC-1 and human islet-derived 1.1B4, 1.1E7 and 1.4E7 cells, incubations were performed at 37°C at various glucose concentrations, values are mean ± sem (n=3), *ρ<0.05, **ρ<0.01, ***ρ<0.001 when compared with 1.1 mM glucose.

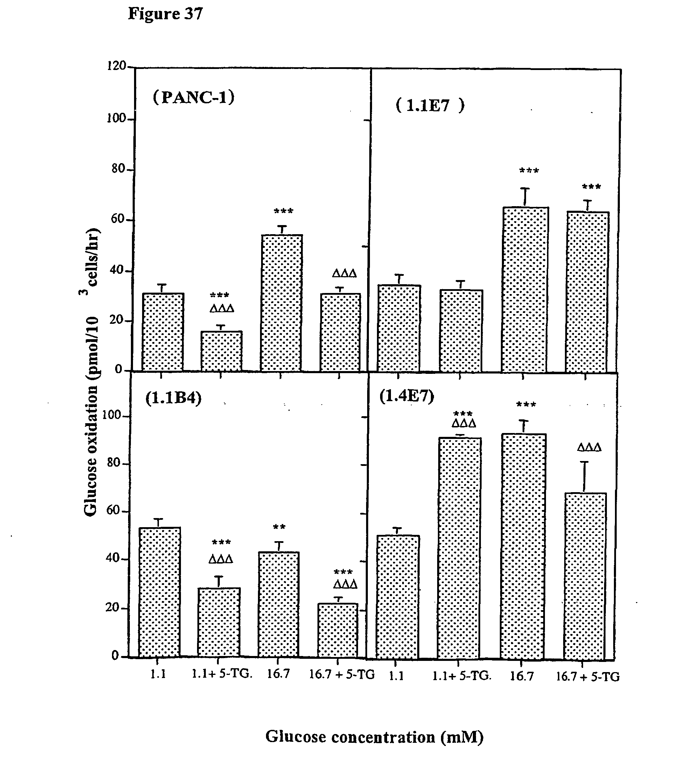

Figure 37 illustrates the effects of 5-thioglucose on glucose oxidation from D-[U-¹⁴C] glucose in PANC-1, human islet-derived 1.1B4, 1.1E7 and 1.4E7 cells, incubations were performed at 37°C at various glucose concentrations, values are mean ± sem (n=3), ΔΔΔρ <0.001 when compared with same glucose concentration in absence of 5-thioglucose, *ρ<0.05, **ρ<0.01, ***ρ<0.001 when compared with 1.1 mM glucose in the absence of 5-thioglucose.

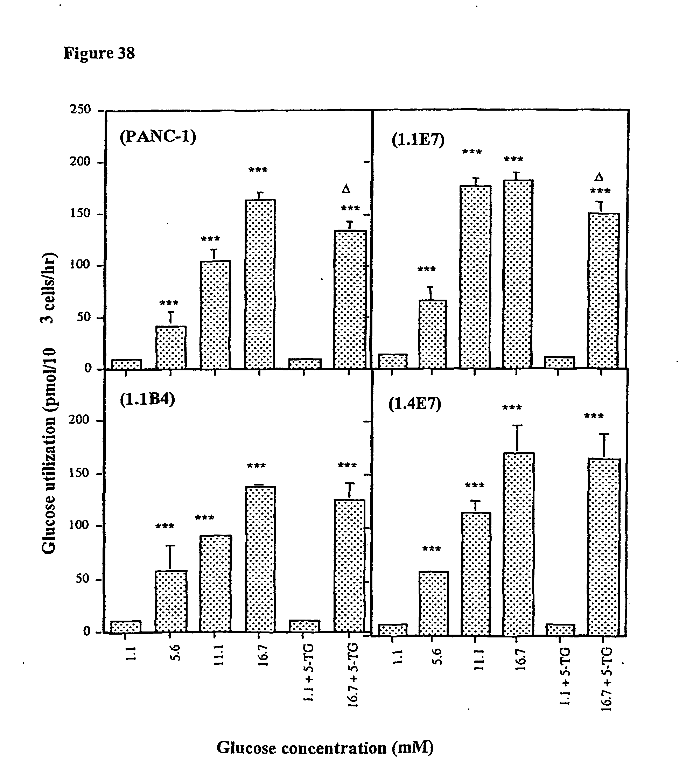

Figure 38 illustrates glucose utilzation from D-[5-³H] glucose in PANC-1, and human islet-derived 1.1B4, 1.1E7 and 1.4E7 cells in the presence or absence of 5-thioglucose, incubations were performed at 37°C at various glucose concentrations, values are mean ± sem (n=3), ***ρ<0.001 when compared with 1.1 mM glucose in the absence of 5-thioglucose, Δρ <0.05 compared with the same glucose concentration in the absence of 5-thioglucose.

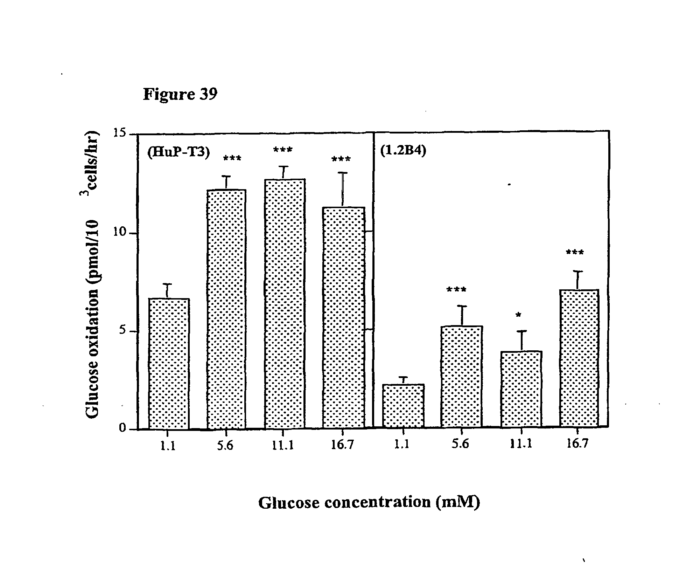

Figure 39 illustrates glucose oxidation from D-[U-¹⁴C]glucose in Hup-T₃ and human islet-derived 1.2B4 cells, incubations were performed at 37°C at various glucose concentrations, values are mean ± sem (n=3), *ρ<0.05, **ρ<0.01, ***ρ<0.001 when compared with 1.1 mM glucose.

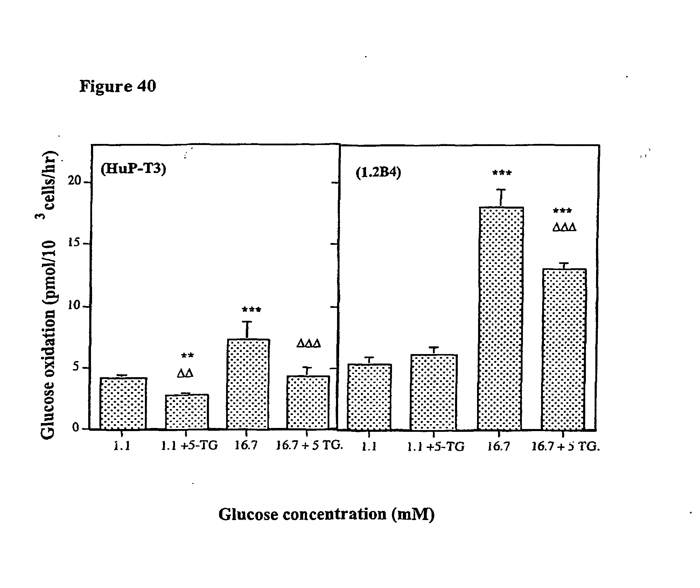

Figure 40 illustrates the effects of 5-thioglucose on glucose oxidation from D-[U-¹⁴C] glucose in Hup-T₃ and human islet-derived 1.2B4 cells, incubations were performed at 37°C at various glucose concentrations, values are mean ± sem (n=3), ΔΔρ <0.01, ΔΔΔρ<0.001 when compared with the same glucose concentration in the absence of 5-thioglucose, **ρ<0.01, ***ρ<0.001 when compared with 1.1 mM glucose in the absence of 5-thioglucose.

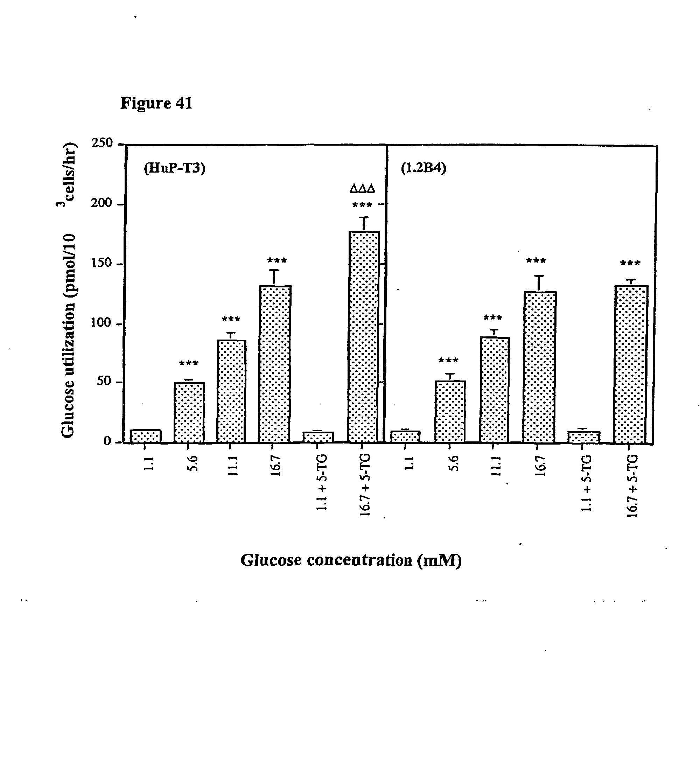

Figure 41 illustrates glucose utilization from D-[5-³H] glucose in Hup-T₃ and human islet-derived 1.2B4 cells in the presence or absence of 5-thioglucose, incubations were performed at 37°C at various glucose concentrations, values are mean ± sem (n=3), ***ρ<0.001 when compared with 1.1 mM glucose in the absence of 5-thioglucose, ΔΔΔρ<0.001 when compared with the same glucose concentration in the absence of 5-thioglucose.

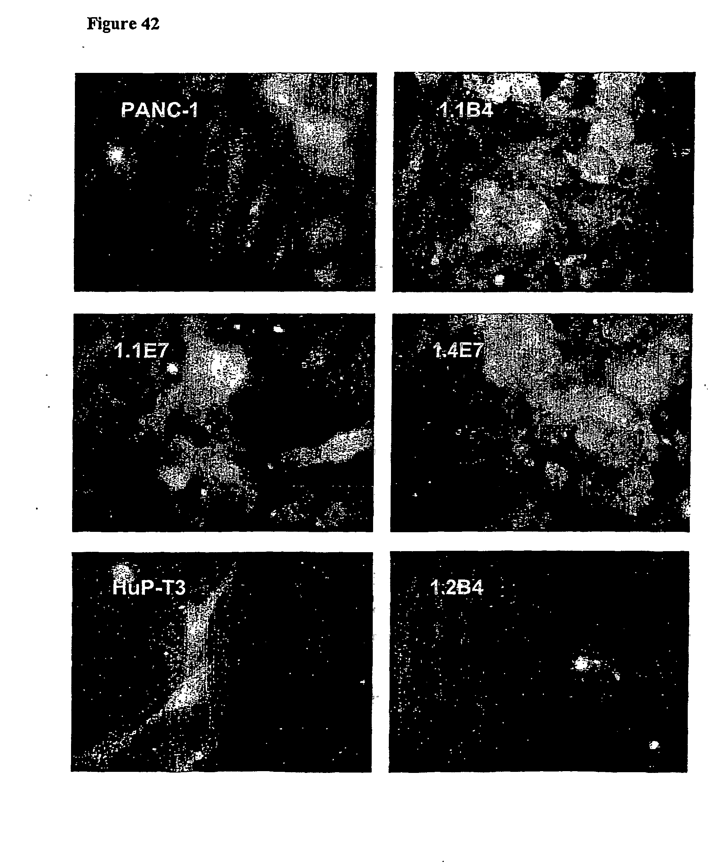

Figure 42 shows immunocytochemical staining for cellular glucokinase (x400 magnification).

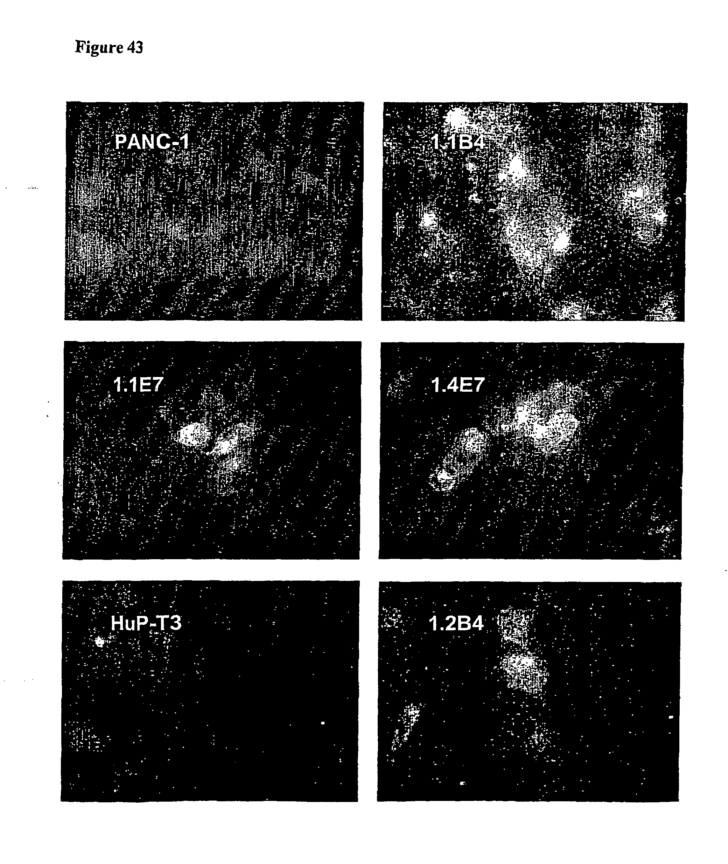

Figure 43 shows immunocytochemical staining for cellular insulin (x400 magnification).

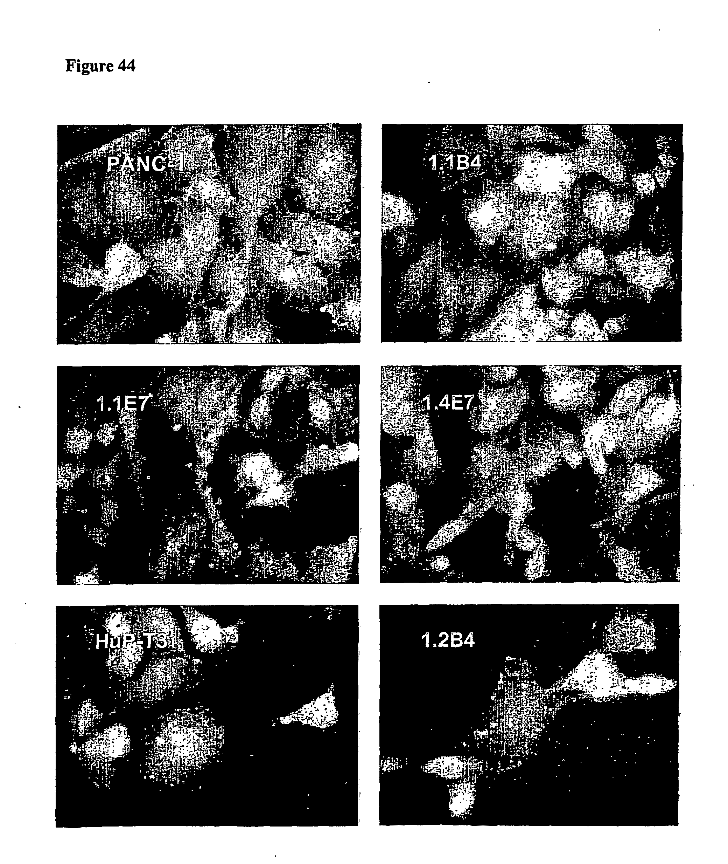

Figure 44 shows immunocytochemical staining for cellular islet amyloid polypeptide (IAPP) (x400 magnification).

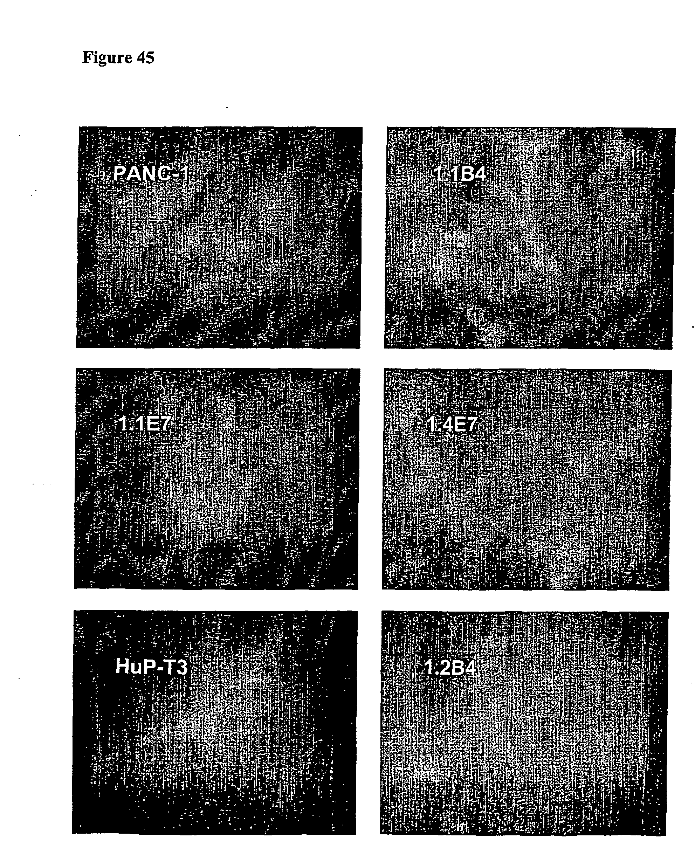

Figure 45 shows immunocytochemical staining for cellular glucagon (x400 magnification).

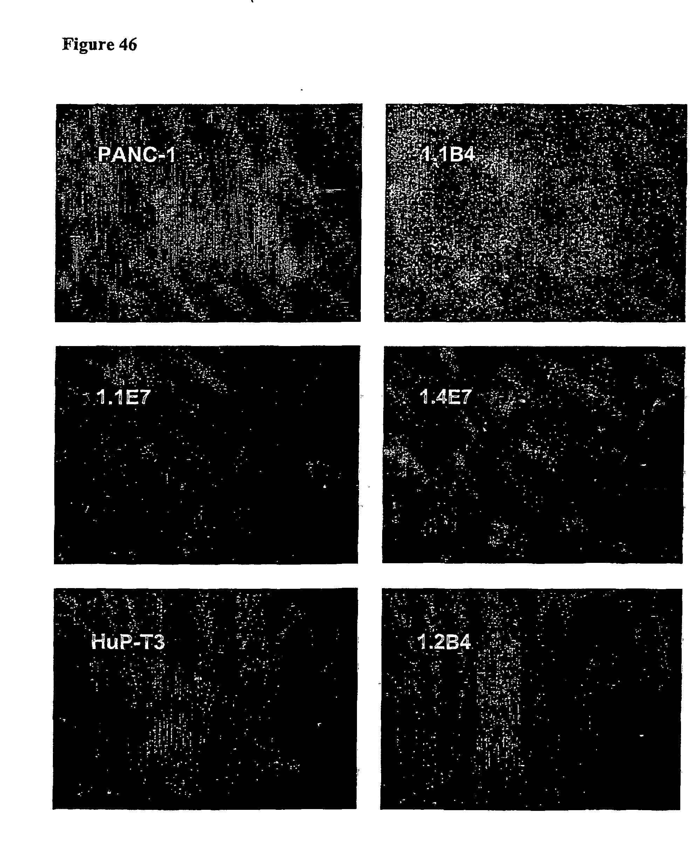

Figure 46 shows immunocytochemical staining for cellular somatostatin (x400 magnification).

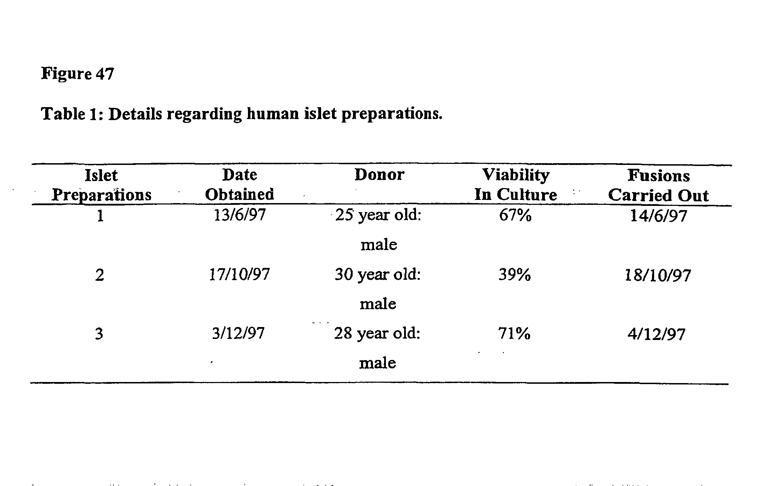

Figure 47 - Table 1 : Details regarding human islet preparations.

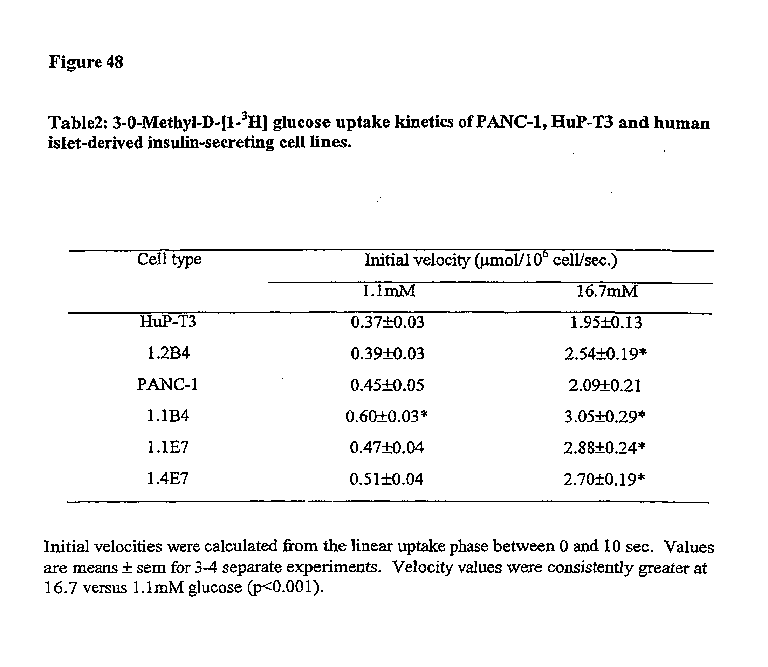

Figure 48 - Table 2 : 3-0-Methyl-D-[1-³H] glucose uptake kinetics of PANC-1, HuP-T3 and human islet-derived insulin-secreting cell lines.

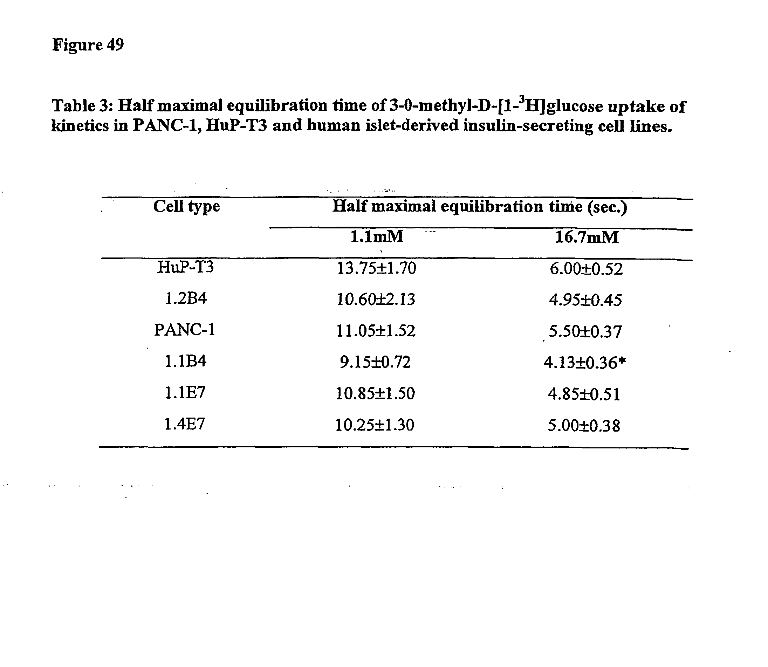

Figure 49 - Table 3 : Half maximal equilibration time of 3-0-methyl-D-[1-³H] glucose uptake of kinetics in PANC-1, HuP-T3 and human islet-derived insulin-secreting cell lines.

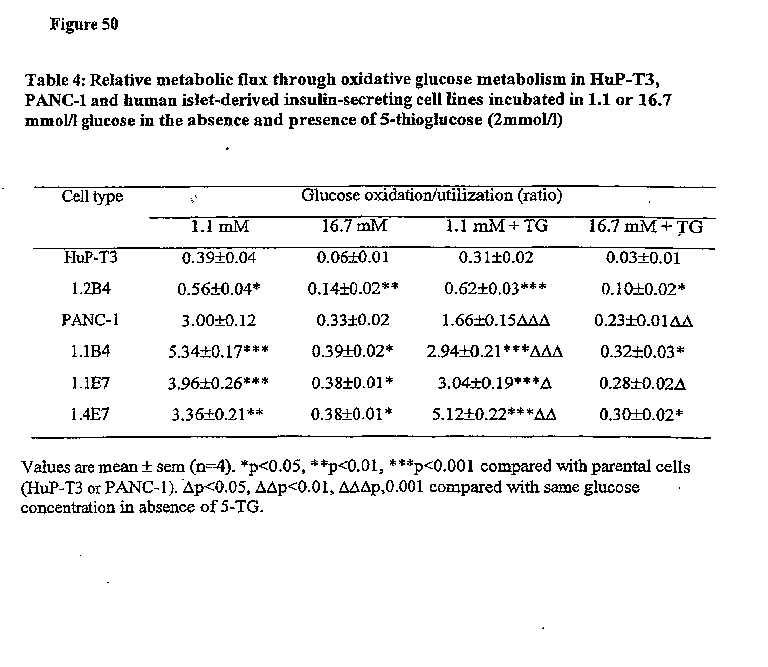

Figure 50 - Table 4 : Relative metabolic flux through oxidative glucose metabolism in HuP-T3, PANC-1 and human islet-derived insulin-secreting cell lines incubated in 1.1 or 16.7 mmol/l glucose in the absence and presence of 5-thioglucose (2 mmol/l).

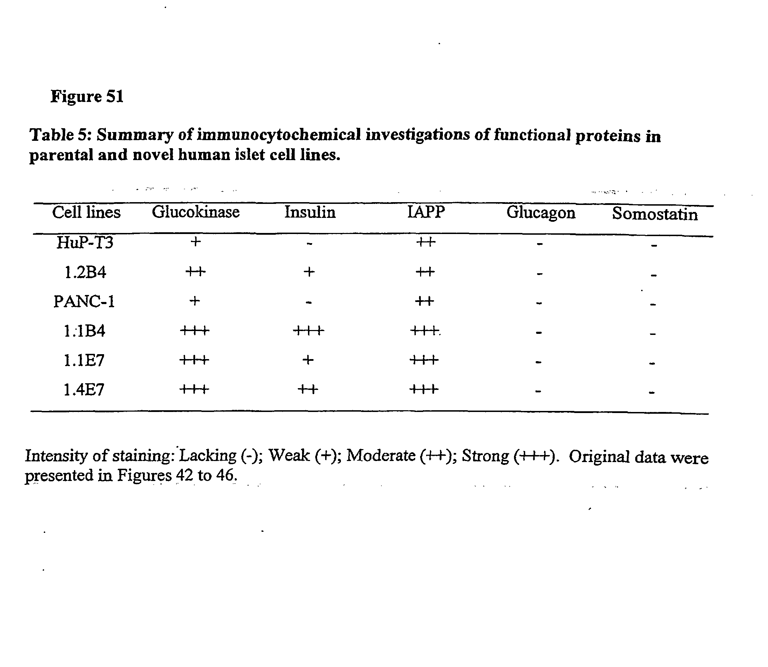

Figure 51 - Table 5 : Summary of immunocytochemical investigation of functional proteins in parental and novel human islet cell lines.

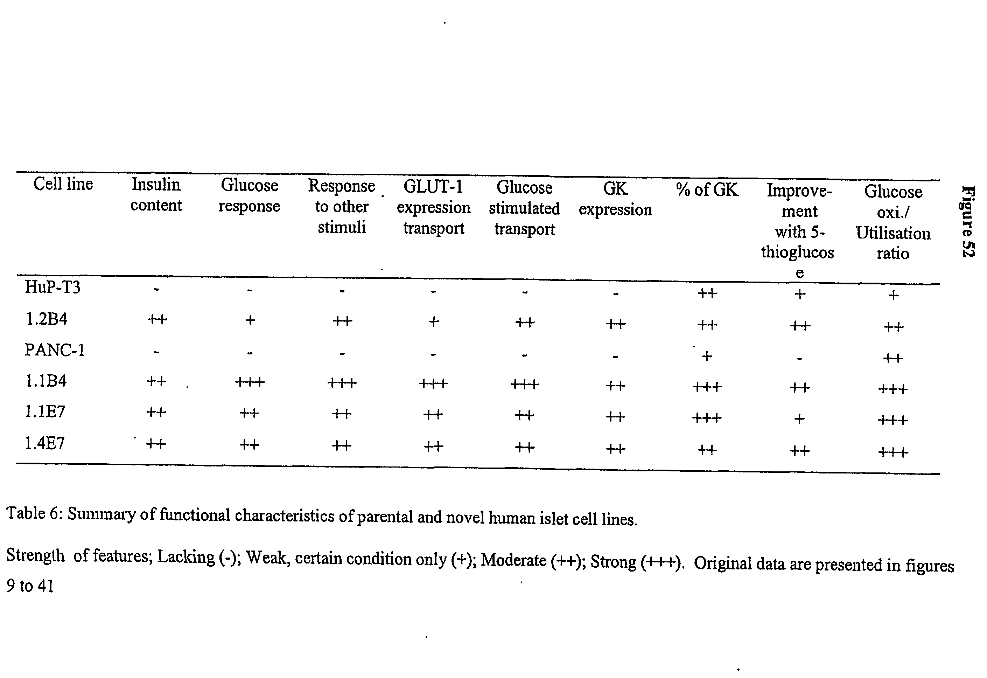

Figure 52 - Table 6 : Summary of functional characteristics of parental and novel human islet cell lines.

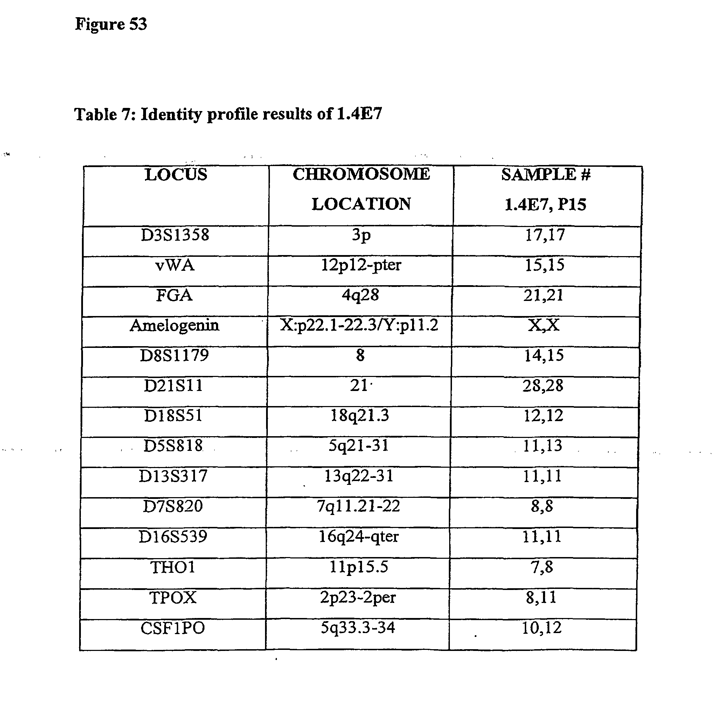

Figure 53 - Table 7 : Identity profie results of 1.4E7.

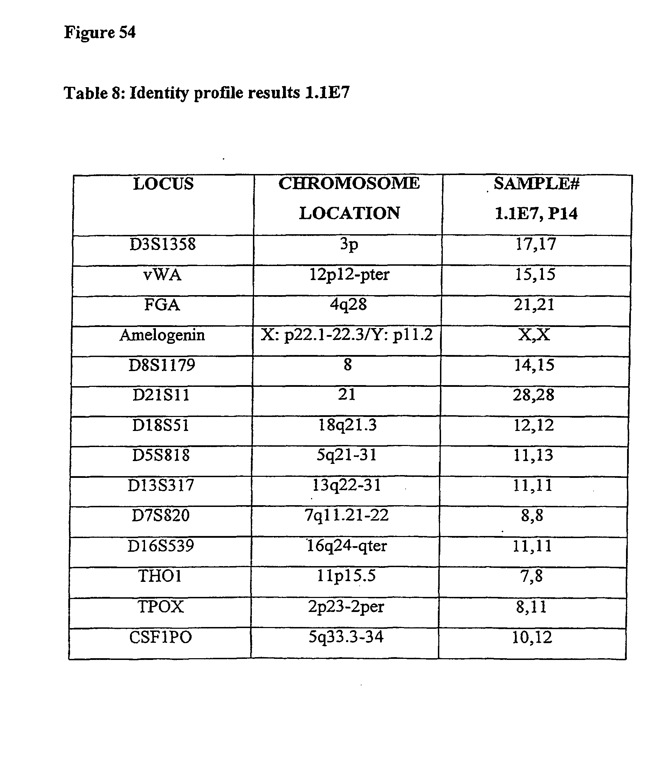

Figure 54 - Table 8 : Identity profile results of 1.1E7.

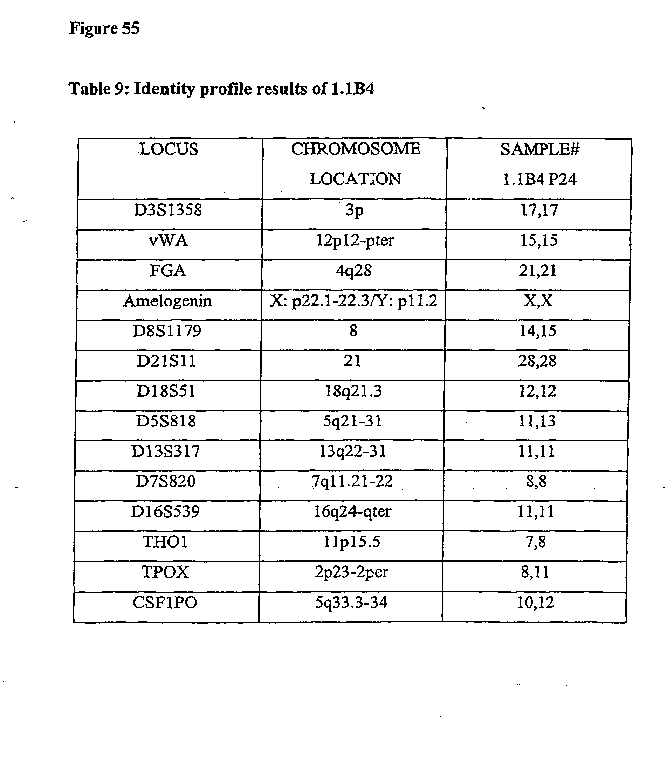

Figure 55 - Table 9 : Identity profile results of 1.1B4.

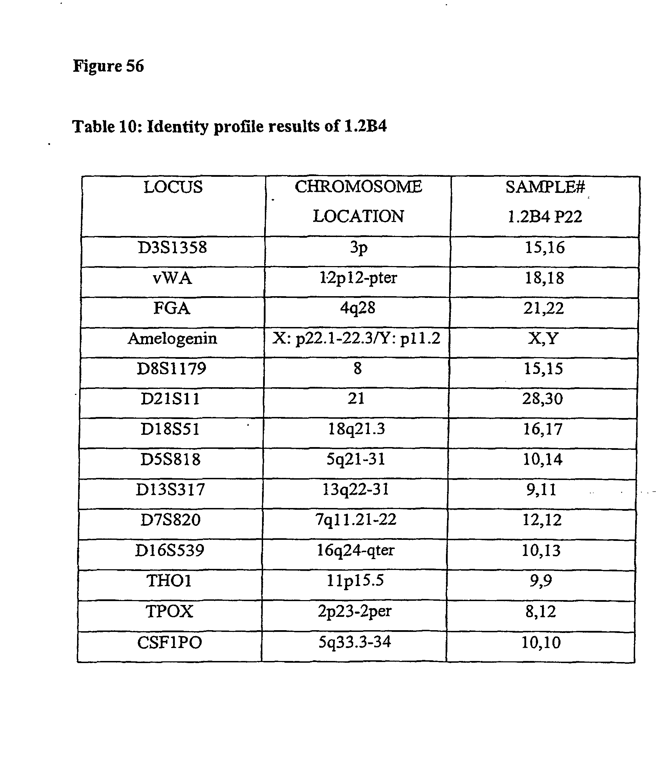

Figure 56 - Tabel 10 : Identity profile results of 1.2B4.

The following embodiments of the present invention describe the use of optimised electrofusion parameters for the generation of novel human insulin-secreting cell lines. Detailed studies are reported on the characteristics and functional properties of the established human pancreatic B-cell clones. Human islets were isolated by Prof. R. Gomis (Barcelona, Spain) and sent in tissue culture medium by overnight courier to Coleraine. Three donor pancreas were employed under local ethical approval. The first batch of 400 islets from a 25 year old male donor were received on 13/6/1997. The second batch of 400 islets from a 30 year old male donor were received on 17/10/1997. The third batch of 300 islets from a 28 year old male were received on 3/12/1997 (Table 1). Islets were disaggregated to single cells and cultured overnight in RPMI 1640 culture medium prior to electrofusion studies.

Immortal PANC-1 and Hup-T₃ cell lines were obtained from ACECC at passage 60 and 52, respectively. PANC-1 is an epithelial cell line derived from a pancreatic carcinoma of ductal origin from a 56 year old Caucasian male (Lieber *et al*., 1975). Hup-T₃ cell line is an epithelial cell line derived from ascites taken from a 66 year old Japanese male (Nishimura *et al*., 1993). The immortal islet-derived human cell fusion partners, TRM-1, HAP5 and B6 were provided by Prof. Hayek (California, USA) and have a passage number of 8, 15 and 10, respectively.

Viability of cells prepared from the three preparations of human islet cells, assessed by trypan blue exclusion after overnight culture, was 67%, 39% and 71%, respectively (Table 1). The islet cells were then resuspended in fusion medium at a density of 2 x 10⁶ cells/ml. The human pancreatic cell lines (PANC-1, Hup-T₃, TRM-1, B6 or HAP6) were harvested, washed twice with fusion medium and resuspended in fusion medium at a density of 2 x 10⁶ cells/ml. Both types of cells were mixed at ratio of 1:1 and 300 µl of cell mixture were pipetted into helical chamber (Biojet, B.Braun Biotech., Germany). This helical chamber was designed closely identical with frame chamber but operated in sterile conditions. The helical chamber was connected with power supply and then exposured in 7 V, 2 MHz of AC field for 30 seconds followed by 60 V, triple pulses of 15 seconds duration times of DC field. The cells were then exposured in 7V, 2 MHz of AC field for 30 seconds as post-fusion alignment.

The fusion mixture was then flushed from the helical chamber with RPMI 1640 culture medium and equally divided into a 24 well plate. Cells were incubated overnight at 37°C. RPMI 1640 culture medium supplemented with HAT (0.1 mmol/l hypoxanthine, 0.4 µmol/l aminopterin and 16.0 µmol/l thymidine) was replaced after 24 hours culture and the cells were maintained in this selective medium for 10 days period. The cell fusion mixture was then cultured in RPMI 1640 culture medium supplemented with HT (0.1 mmol/l hypoxanthine and 16.0 µmol/l thymidine) and cells were maintained in this medium until colonies of hybrids were seen under microscopic observation.

Colonies of hybrid cells normally appeared after 10 to 20 days of fusion as observed under an inverted microscope. Colonies were maintained for another 10-20 days before medium was replaced for 24 hours before screening. A single screening procedure was used to select hybrid insulin-secreting cells from fusion mixture. In order to determine accurately the insulin concentration in the samples, controls were prepared using culture media alone or media from 24 hours culture of parental cells. This approach allowed automatic correction for the possible presence of insulin or interfering substances in the culture medium.

Aliquots of 200 µl culture medium were removed in duplicate and the insulin concentration released into culture medium was measured by radioimmunoassay as described below. Wells containing hybrid insulin-secreting cells were selected and cells from each well were harvested and divided into 24 well plate for the next screening step.

The novel human insulin-secreting cell lines were cultured in RPMI 1640 culture medium supplemented with 10% (v/v) foetal bovine serum and antibiotics (100µg/ml penicilin and 0.1 g/l streptomycin). The cells were maintained under culture condition (37°C, 5% CO₂ and 95% air) before the cells were used for experiments. Where appropriate cells were cryopreserved and stored.

Cells were harvested with the aid of trypsin and then resuspended in tissue culture medium. Cells were seeded at a density of 5 x 10⁵ cells per well in 6 well plate and allowed to attach overnight in the 37°C incubator. Following the removal of tissue culture medium, the growing cell monolayers were then viewed using a phase contrast microscope (Zeiss, Germany) and images were captured by Imagedok version 2.0 software package (Kinetic Imaging Ltd, UK).

Acute static incubations at 37°C were performed using monolayers of insulin-secreting cells. Twenty four hours prior to experimentation, the cells were harvested and then resuspended in RPMI-1640 tissue culture medium. Approximately 2.5 x 10⁵ cells were then seeded in each well of 24-well multiplates (Iwaki Glass, Japan). After culture at 37°C and attachment of cells overnight, the culture medium was removed and 1ml of KRBB buffer (pH 7.4) supplemented with 1% (w/v) bovine serum albumin (BSA) and 1.1 mmol/l glucose, was carefully added to each well. The cells were incubated at 37°C for 40 minutes, after which the buffer was removed and replaced with 0.5 ml KRBB test buffer. The KRBB test buffer was supplemented with 0.5 g/l (w/v) bovine serum albumin (BSA) and 1.1 mmol/l glucose along with the established modulators of pancreatic B-cell function. After 60 min incubation at 37°C, a 450 µl aliquot of KRBB test buffer was removed from each well into 2ml polypropylene tube (Lip Plastics, England) and centrifuged at 900 rpm for 5 minutes at 4°C. The supernatant was then collected from each tube and 200µl aliquots were then stored at -20°C for subsequent insulin determination by radioimmunoassay.

After harvesting, the cells were resuspended in tissue culture medium, seeded at a density of 2.5 x 10⁵ cells per well, and allowed to attach overnight to form monolayers in 24 well multiplates (Iwaki Glass, Japan). The culture medium was then completely removed and 500 µl of acid-ethanol solution (1.5% (v/v) HCl, 75% (v/v) ethanol, 23.5% (v/v) H₂O) was added. The cells were disrupted with the aid of a pasteur pipette and incubated overnight at 4°C prior to centrifugation at 900 rpm for 5 minute. Supernatants were stored at - 20°C for subsequent determination of cellular insulin content by radioimmunoassay.

Insulin was measured by modified dextran-charcoal radioimmunoassay (Albano *et al*., 1972) as described by Flatt and Bailey (1981). Working radioimmunoassay phosphate buffer (40 mmol/l sodium phosphate buffer, pH 7.4 supplemented with 0.5% (w/v) bovine serum albumin) was used to dilute human insulin standards, insulin antibody, ¹²⁵I-labelled insulin and samples as appropriate. Human insulin standards were prepared over a range of 2-fold serial dilutions from 5.0 ng/ml to 0.009 ng/ml using Human insulin standard (Sigma Chemical, UK). 200µl of the standard (pipetted in triplicate) or unknown sample (pipetted in duplicate) was mixed with 100µl of guinea pig anti-bovine insulin antiserum at around 1: 100,000 dilution) and incubated for 24 hours at 4°C prior to the addition of 100 µl of ¹²⁵I-insulin (approx. 100 pg.). This antiserum was specificaly selected for high affinity with human insulin standard.

After a further 24 hours incubation at 4°C, 1ml of a 5% (w/v) charcoal coated with 1:10 dextran T-70 in sodium phosphate buffer (40 mmol/l, pH 7.4) was added to each tube. After 20 minutes incubation (4°C), each tube was centrifuged at 2500 rpm for 20 minutes (4°C). The supernatant was decanted and the counts of ¹²⁵I-insulin bound to charcoal were recorded using a gamma counter (1261 Multigamma Counter, LKB Wallac, Turku, Finland) linked to a computer (Olivetti PCS 286). Counts bound to antibody (average total counts minus counts bound to charcoal) are inversely proportional to the concentration of insulin in the standard or unknown sample. The unknown concentration was determined by means of the standard curve, constructed from the known rat insulin standard using a spline curve fitting algorithim. This assay gives 10%, 50% and 90% fall in bond counts an addition of 19 pg/ml, 250 pg/ml and 2.5 ng/ml human insulin, respectively.

Protein concentration were determined using a modification of the method described by Bradford (1979). The Bio-Rad protein assay is a dye binding assay based on the differential absorbance change of Coomassie Brilliant Blue G250 under acidic conditions in response to various concentrations of protein. The Bio-Rad protein assay reagent was diluted with distilled water and glacial acetic acid in the ratio 4:5:1 respectively. This was then filtered through Whatman No. 1 grade filter prior to use. Known standards were prepared in the range of 1-20 µg/µl of bovine serum albumin in distilled water. The reagent (200µl) and 25µl sample (standard protein or unknown) were then mixed several times times by gentle inversion in microtiter plate. After a period of 30 minutes, the absorbance at 595 nm was measured on a microtiter plate reader (Thermo, UK). A standard curve was produced from the BSA standard each time the assay was performed, and the sample protein concentration determined.

The cell lines (parental and clonal) were harvested (approximately 5 x 10⁷ cell per preparation) and resuspended in phosphate buffer saline, pH 7.4. Following 6 cycles of sonication (10 seconds/cycle) using a Soniprep 150 (MSE, UK), the cell lysates were centrifuged for 10 minutes at 10,000 rpm (Beckman Ultracentrifuge, USA). The supernatant was collected and centrifuged for another 1 hour at 45,000 rpm. The membrane pellets obtained were resuspended in PBS and protein membrane concentration was measured by the Bio-Rad assay as described above.

Insulin-secreting cells were harvested (approximately 4 x 10⁷ cells per preparation) and resuspended in ice-cold sonication medium (20 mmol/l Hepes, 210 mmol/l mannitol, 70 mmol/l sucrose, adjusted to pH 7.4 using KOH, supplemented with 1 mmol/l dithiotreitol and 5% glycerol). The cells were then sonicated (Soniprep 150, MSE, UK) for 30 seconds at 4°C. A soluble cytoplasmic fraction was obtained by three successive centrifugation steps at 4°C of 5000 rpm, 10,000 rpm, and 45,000 rpm, respectively (Lenzen *et al*., 1985). Glucokinase protein concentration was measured by the Bio-Rad assay as described above. Glucose phosphorylating enzyme activity was determined using the Varian Cary 1 UV/VIS spectrophotometer (Philips, UK)), recording the formation of NADPH. Rates of glucose phosphorylation in the 45,000 rpm soluble cytoplasmic fractions were assayed at 37°C (pH 7.4) by recording the increase in absorbance at 340 nm in 500 ul mixture containing 20 mmol/l Hepes (adjusted to pH 7.4 with KOH), 125 mmol/l KCl, 7.5 mmol/l MgCl₂, 5 mmol/l ATP, 0.5 mmol/l NADP, 700 U/I Glucose-6-phosphate dehydrogenase, 10 U/I 6-P-gluconate dehydrogenase plus cytoplasmic supernatant (50 µl) from each aliquot of cells. Hexokinase activity was assayed in the presence of 1 mmol/l D-glucose and subtracted from the total activity recorded at 100 mmol/l D-glucose to give glucokinase activity (Lenzen *et al*., 1985). One unit of enzyme activity was defined as 1 µmole of glucose-6-phosphate formed from glucose and ATP per minute at 37°C. Protein determination was performed on the cytoplasmic supernatant using the Bio-Rad assay, as previously described.

Insulin-secreting cells were seeded at a concentration of 2 x 10⁵ cells/well in 35 mm² tissue culture dishes (Nunclon, Denmark) and cultured for 24 hours at 37°C. Cells were given two rapid washes in phosphate buffer saline and then preincubated for 30 minutes at 37°C in KRBB supplemented with 1.1 mmol/l glucose and 0.1% BSA. Cells were then incubated with 0.5ml of KRBB supplemented with 3-O-methyl-D-[1-³H] glucose (Amersham Pharmacia, USA) for 0, 5, 10, 20, 60, 120, 240 and 360 seconds in 37°C. Tested concentrations of 1 µCi/ml 3-O-methyl-D-[1-³H] glucose were used in KRBB containing 1.1 and 16.7 mmol/l D-glucose. During the incubation, plates were stood on a hot plate heated to 37°C. Uptake of tritiated with 3-O-methyl-D-[1-H³] glucose was terminated with 3 rapid washes using 0.5ml ice-cold KRBB containing 100 mmol/l glucose. Cells were then lysed with 1ml 0.5% sodium dodecyl sulphate (SDS) and left at room temperature for 20 minutes. Scintillation liquid (HiSafe, BDH) was added (3ml) to the lysate and mixed well. The vials were left to stand for 3 hours in the dark to allow any bubbles to rise to the surface. The counts of radioactivity in the lyste was quantified by liquid scintillation counting (Wallac 1409 liquid scintillation counter, Finland).

A modification of the method developed by Keen *et al*. (1963) was used to determine oxidative glucose metabolism to CO₂. In principle, the method involves incubation of cells in specially designed glass cups suspended inside standard counting vials with an airtight rubber stopper. A disc of filter paper (Whatman No. 1) was placed on the base of the counting vial. For each sample, 2.0 x 10⁵ cells were incubated in the presence of either 1.1, 5.6, 11.1, 16.7 mmol/l glucose and D-[U-¹⁴C] glucose to give a final concentration of 0.25µCi (40µl total volume). Effect of 2 mmol/l 5-thioglucose were also tested in 1.1 and 16.7 mmol/l glucose Blanks were set up where the cells were omitted and replaced with KRBB to give a final concentration of either 1.1 or 16.7 mmol/l glucose. Standards consisted of ¹⁴C-labelled glucose (0.5µCi) added to the filter paper at the base of the counting vial. The glass vials were sealed with the rubber caps and placed at 37°C for one hour. The reaction was stopped by the addition of 0.2M HCl (50µl) injected through the rubber cap directly into the centre well using a Hamilton syringe. Phenylethylamine (PEA) was added (100µl) through the sealed lid onto the filter paper at the base of the vial. Care was taken to avoid contamination of the centre well with PEA. Vials were then placed at 37°C for one hour. The rubber caps and centre wells were removed and 5ml of liquid scintillation fluid (BDH) added to each vial on top of the filter paper. Vials were capped and ¹⁴C- glucose labelled radioactivity determined by scintillation counter.

Experiments were performed in specially designed glass cups suspended inside standard counting vials with an airtight stopper. For each sample, 2.0 x 10⁵ cells were incubated in the presence of either 1.1, 5.6, 11.1 or 16.7 mmol/l glucose and D-[5-³H] glucose to give a final concentration of 0.25µCi (40µl total volume). Effects of 2 mmol/l 5-thioglucose were also tested in 1.1 and 16.7 mmol/l glucose. Blanks were set up where the cells were omitted and replaced with KRBB to give a final concentration of either 1.1 or 16.7 mmol/l glucose. Standards consisted of D-[5-³H] glucose (0.5µCi) added to the base of the counting vial. The glass vials were sealed with the rubber caps and placed at 37°C for one hour. The reaction was stopped by the addition of 0.2M HCl (50µl) injected through the rubber cap directly into the centre well using a Hamilton syringe. Sterile deionized water was added (500µl) through the sealed lid into the base of the vial. Vials were then placed at 37°C for a 15 hour. The rubber caps and centre wells were removed and 5ml of liquid scintillation fluid (BDH) added to each vial on top of the filter paper. Vials were capped and ³H-glucose labelled radioactivity determined by scintillation counter.

### SDS-PAGE Gel Preparation:

The following reagents and buffer were prepared:

| | |
|---|---|
| Lower Gel Buffer | 1.5mmol/l Tris-HCl, pH 8.8 |
| Upper Gel Buffer | 0.5 mmol/l Tris-HCl, pH 6.8 |
| Electrode Buffer | 25 mmol/l Tris, 192 mmol/l Glycine and 0.1% (w/v) SDS |
| Sample Buffer | 125 mmol/l Tris-HCl, pH 6.8 containing 4.6% (w/v) sodium dodecyl sulphate (SDS), 30% (v/v) glycerol, 0.002% (w/v) bromophenol blue and 10% (v/v) 2β-mercaptoethanol. |
| Acrylamide | 30% (w/v) acrylamide. |

A vertical slab mini-gel apparatus (Bio-Rad) was used for this analysis. The gel was cast in a glass cassette, with perspex slide held together in the gel apparatus. The dimensions of the gel were 80mm x 73mm x 1mm. The glass front and back were washed with decon detergent, rinsed with tap water and then distilled water and finally cleaned with acetone and left to air dry. The cassette was assembled and clamped into a vertical position.

The lower gel was prepared by mixing the following reagents in a 25ml universal tube: lower gel buffer (2.5ml), distilled water (4.2ml), SDS 10% (w/v) (100µl), acrylamide (3.3ml) and ammonium persulfate 10% (w/v) (50µl). Polymerisation was initiated by the addition of 5µl of N,N,N'N' -tetramethylethylenediamine (TEMED). The solution was poured into the cassette to a height of 10 cm. A thin layer of distilled water was placed on top of the gel solution to ensure a flat interface between the lower and upper gel. Polymerisation of the lower gel was complete in 60-90 minutes, at which time the layer of water was removed.

The upper stacking gel was then prepared by mixing the following reagents in a 25ml universal tube: upper gel buffer (1.25ml), distilled water (3.05ml), SDS 10% (w/v) (50µl), acrylamide (670µl) and ammonium persulfate 10% (w/v) (25µl). Polymerisation was initiated by the addition of 5µl of N,N,N'N' - tetramethyl-ethylenediamine TEMED and the upper stacking gel poured into the cassette above the lower gel. A perspex comb was introduced into the upper gel to form the sample wells and after approximately 30 minutes the gel had polymerised. Protein content was determined using the Bradford method as described previously. The samples (GLUT-1 and glucokinase protein preparations) were then boiled for 5 minutes in a water bath to denature the protein prior to loading into the sample wells. The comb was subsequently removed forming sample wells into which protein sample (containing 100µg protein) and protein biotinylated molecular weight marker (Amersham, UK) were loaded. The cassette was then placed into the electrophoresis tank. The tank was then assembled and electrode buffer was placed in the upper and lower reservoirs.

Electrophoresis was performed immediately following sample loading at a constant current of 40mA, 120V using a Pharmacia EPS500/400 power pack (Pharmacia, USA) until the bromophenol blue band had migrated to the lower edge of the lower running gel, the current was then switched off. The cassette was then removed from the.electrophoresis tank and the glass plates separated, the lower gel was separated from the upper stacking gel and used for the transfer of proteins onto nitrocellulose for Western blotting analysis.

The following buffers were prepared for Western blotting and detection. The transfer buffer comprised Tris (20 mmol/l), glycine (150 mmol/l) and 15% (v/v) of methanol. The wash buffer comprised phosphate buffer saline (PBS) supplemented with 0.2% (v/v) polyoxyethylene sorbitan monolaurate (Tween-20). The blocking buffer comprised PBS supplemented with dried skimmed milk 2.5%(w/v).

For Western blotting, the lower running gel was removed from the cassette and placed in transfer buffer (30ml) for 30 minutes to allow the buffer to permeate the gel and replace the electrode buffer. During this time the gel shrank as it equilibrated with the transfer buffer. After equilibration, the gel was removed and placed into the transfer cassette. The cassette was prepared under transfer buffer and all the air bubbles removed by rolling with a glass test tube.

Once assembled, the cassette was placed into a transfer tank with the nitrocellulose sheet closest to the positive electrode. The transfer was performed at a constant current of 350mA, 100V for 90 minutes using Bio-rad Transblot System (Richmond, CA, USA) according to the method of Towbin *et al*. (1979). The cassette was opened and the nitrocellulose membrane placed into 25ml blocking buffer and rocked for one hours followed by incubation overnight at 4°C.

The nitrocellulose was washed in PBS-T solution five times for five minutes each, followed incubation for 1 hour with the primary GLUT-1 antibody (Santa Cruz Biotechnology, CA, USA) or glucokinase antibody (S. Lenzen, Hanover, Germany) diluted (1:1000) in PBS. The washing steps were repeated before a one hour incubation of the membrane with horseradish peroxidase labelled antibody (Amersham, UK) diluted (1: 1000) in PBS. The membranes were then washed and incubated for one hour in streptavidin- HRP (horse radish peroxidase) conjugate diluted (1:1500) in PBS. After final washing steps (consisting of 3 washes of 5 minutes duration in wash buffer, followed by 2 washes in PBS alone) the signal of protein expression was visualised using the Amersham enhanced chemiluminescence (ECL) detection system. Basically, this system works on the HRP/H₂O₂ catalysed oxidation of luminol in alkaline conditions, in the presence of chemical enhancers such as phenols. The light produced by this enhanced chemiluminescent reaction can be detected by the short exposure (30 seconds) to blue-light sensitive autoradiography film, Hyperfilm ECL (Amersham, UK).

Prior to immunohistochemical studies, each cell line was plated onto 'supercell' incubation slides containing 8 separate plastic chambers per slide (BDH/Merck, UK.) and placed in culture at 37°C overnight to allow the cells to adhere to the slides. The plastic chambers holding the tissue culture medium were carefully removed and the slides briefly washed in phosphate buffered saline (PBS, Oxoid) before fixation in ice-cold 4% paraformaldehyde (BDH/Merck)/PBS for 20 min. After washing with PBS, the cells were permeabilised in a solution of 0.3% triton X-100 (Sigma Chemicals) for 15 min. Further washes in PBS were followed by blocking the cells with 2% normal goat serum (Vector Laboratories, CA, USA)/1% BSA/PBS for 90 min at room temp. Incubation with the primary antibody (insulin and glucagon guinea pig anti-porcine antisera from Prof. PR. Flatt, used at a dilution of 1:1000; Glut 1 affinity purified goat polyclonal antibody from Santa Cruz biotechnology CA, USA, used at a dilution of 1;100; human somatostatin and amylin rabbit antisera from Penninsula Laboratories Inc, Belmont, CA, USA, all used at a dilution of 1:200; Glucokinase rat polyclonal antisera, gift from Prof. Sigurd Lenzen (Hannover, Germany), used at a dilution of 1:1000) was carried out for 1 hour at 37°C, all dilutions were made in 0.2% normal goat serum/0.1% BSA/PBS. After thorough washing in PBS cells were incubated with fluorescein anti-rabbit IgG (H+L) made in goat (Vector Laboratories, CA, USA) at a dilution of 1:40 in PBS, for 45 min at 37°C. Slides were given 5 x 5 min washes in PBS and mounted under a glass coverslip in a solution of 50% glycerol (Sigma Chemicals)/50% PBS. Immunohistochemical staining was visualised using a Nikon OP-5 microscope and images recorded using the PC Imagedok program.

The procedure for screening is summarized in Figure 1. Hybrids cells produced by electrofusion were selected through a screening procedure based on a high level of insulin output. Basically, for one electrofusion experiment the cell fusion mixture was divided into 24 wells and measurement of insulin output was taken over the final 24 hours of the 30 to 35 day culture period. Initial experiments were performed using the immortal islet-derived human cell fusion partners, TRM-1, HAP5 and B6 provided by Professor Hayek (California, USA). The results of 2 fusion experiments between HAT-sensitive TRM-1 cells and human islet cells are summarised in Figure 2. Post-fusion selection revealed surviving cell hybrids in 24 wells, with 13 wells containing hybrids secreting insulin at 40 days. Attempts to clone the insulin-releasing hybrids were thwarted by rapidly declining growth rate indicating that the TRM-1 cell line was not fully immortalised. Similar results were obtained using HAP5 and B6 cells (data not shown), at least illustrating an expected yield of about 10 human hybrid cells per fusion. Two fusion with HAP5 cells yielded 12 and 11 hybrids of which 4 and 6 produced insulin at 12 days. The two B6 cells fusions yielded 15 and 13 hybrids with 5 and 3 releasing insulin at 6 days.

In view of problem with the initially adopted fusion partners (TRM-1, HAP5 and B6), attention was focussed on use of human pancreatic adenocarcinoma cells, PANC-1 and Hup-T₃, obtained from European Collection of Animal Cell Culture (ECACC). HAT-sensitive clones and electrofusion parameters for these cell lines were established. The screening procedure which involved fusion between PANC-1 and human islets cells revealed 5 positive hybrid colonies hybrids from the total of 25 hybrid colonies (Figure 1, 3A, 4A). Cells from well A and well B were selected for further evaluation. After a series of cloning steps, the clones 1.1B4, 1.1E7 and 1.4E7 were isolated (Figure 3B-4B). The other 16 insulin-releasing clones were cryopreserved but not studied further.

Cell line 1.1E7 was produced by fusion of human islet cells with an established cell line PANC-1, 1.1E7, P27, 07052001.

Cell line 1.4E7 was produced by fusion of human islet cells with an established cell line PANC-1, 1.4E7 P29, 07112001.

Similar screening procedures were taken for fusion between Hup-T₃ cell lines and human islet cells (Figure 1). The first screening after 30 days of fusion showed 13 wells from a total of 48 wells contained hybrid colonies, 4 of which were insulin-positive hybrids (Figure 5A). Hybrid cells from well C were selected for a series of cloning steps, after which the insulin-releasing clone 1.2B4 was isolated (Figure 5B). The other 7 insulin-releasing clones were cryopreserved.

Cell line 1.2B4 was produced by fusion of human islet cells with an established cell line Hup-T₃, 1.2B4, P27, 07102001.

As shown in Figure 6-8, parental PANC-1, Hup-T₃ and the derived human islet cell clones grew as monolayers in tissue culture. When confluent each cell line took on a pavemental pattern as is routinely observed with epitheliod cell lines. The human islet hybrid cell lines showed consistent growth patterns with approximate doubling times of 23 ± 1.7 hours for 1.1B4 cells, 23 ± 1.9 for 1.1E7 cells, 26 ± 1.5 hours for 1.4E7 cells and 36 ± 2.1 hours for 1.2B4 cells (n=4). All of the islet-derived cell lines maintained their gross morphological appearance and doubling times at passage 40. The approximate doubling times on non-fused PANC-1and Hup-T₃ were 21 ± 1.6 and 30 ± 2.3 hours (n=4), respectively.

As shown in Figure 9, the cellular insulin content (mean ± sem, n = 12) of 1.1B4, 1.1E7, 1.4E7 and 1.2B4 cells at passage 17 was 0.394 ± 0.019, 0.354 ± 0.021, 0.351 ± 0.023 and 0.363 ± .017 ng/10⁶ cells respectively. At passage 40, there was no significant change in the cellular insulin content of each cell line. Insulin contents of 0.408 ± 0.024, 0.314 ± 0.018, 0.361 ± 0.02 and 0.306 ± 0.02 ng/10⁶ cells were observed for 1.1B4, 1.1E7, 1.4E7 and 1.2B4 cells, respectively. PANC-1 and Hup-T₃ cells did not contain immunoreactive insulin.

The effects of glucose on insulin secretion from each of the 4 novel human islet cell lines cells are shown in Figure 10. Each cell line showed a different pattern and magnitude of responsiveness to acute glucose concentrations exposure. 1.1B4 cells showed a stepwise 2.3-fold insulin-secretory response to increasing glucose concentration over the range 0 mmol/l to 16.7 mmol/l glucose, with a threshold for insulin release at 5.6 mmol/l glucose. Both 1.1E7 and 1.4E7 cells showed maximal 1.6 and 1.5-fold insulin-secretory response to glucose at 11.1 mmol/l and 5.6 mmol/l glucose, respectively. In contrast Hup-T₃-derived 1.2B4 cells, showed no significant insulin response to glucose over the range from 0 mmol/l to 16.7 mmol/l glucose.

The insulin release from human islet-derived clones in 1.1 mmol/l glucose as a % of the cellular insulin content was shown in Figure 11. Basal insulin secretion from each of the human islet-derived.clones represented between 9% - 11% of the cellular insulin contents.

Figure 12 shows insulin secretory response to glucose from each of the four cell lines in the presence of isobutylmethylxanthine (IBMX). Inclusion of 200 µmol/IBMX in the test buffer with various glucose concentrations from 0 mmol/l to 16.7 mmol/l greatly enhanced the stimulatory effect of glucose on 1.1B4, 1.1E7 and 1.4E7 cells. As shown in Figure 13-15, the secretory response was significantly increased by 20 to 100% to 1.3-2 fold stimulation (*p*<0.001) of 1.1B4, 1.1E7 and 1.4E7 cells. However, inclusion of IBMX did not significantly affect insulin released from 1.2B4 (Figure 16).

The effects of 5-thioglucose on the acute glucose responsiveness of the human cell lines are shown in Figure 17. Responsiveness of all four types of cell to increasing of glucose concentrations was improved by the inclusion of 5-thioglucose in the incubation buffer. Although 5-thioglucose did not appear to enhance the stimulating effect of glucose, it greatly reduced basal insulin release and caused a generalised right shift in the glucose responsiveness of 1.1B4, 1.1E7 and 1.2B4 cell lines toward more physiological concentrations. Comparison of absolute rates of insulin release indicated that inclusion of 5-thioglucose significantly lowered insulin output from 1.1B4, 1.1E7 and 1.2B4 by 20 - 50% (*p*<0.05 to *p*<0.001; Figures 18,19,21). Of the cell lines tested, 1.4E7 cells were least affected by inclusion of 5-thioglucose in the incubations (Figure 19).

As shown in Figures 22-25, the effects of a range of nutrient and pharmaceutical secretagogues were tested at two diferent glucose concentrations (5.6 mmol/l and 11.1 mmol/l ). Each cell line showed differences in both the magnitude and pattern of responsiveness.

The secretory responses of 1.1B4 cells to a range of modulators tested at 5.6 mmol/l glucose are shown in Figure 22A. All stimulators, including glyceraldehyde, leucine, KIC, arginine, alanine, tolbutamide and a depolarising concentration of KCl, significantly (*p*<0.05 to *p*<0.001) increased insulin secretion by 1.2-2 fold. As shown in Figure 22B, increasing the glucose concentration from 5.6 mmol/l to 11.1 mmol/l significantly increased (*p*<0.01 to *p*<0.001) the effects of leucine, arginine and tolbutamide by 30, 17 and 20%, respectively.

The insulin secretory responses of 1.1E7 cells to the same range of stimulators are shown in Figure 23. At 5.6 mmol/l glucose, glyceraldehyde, arginine and KCl evoked significant 1.2-1.8 fold insulin secretory responses (*p*<0.05 to *p*<0.001; Figure 23A). However when tested at the higher glucose concentration of 11.1 mmol/l glucose, all stimulators elicited a significant increase insulin output (*p*< 0.05 to *p*<0.001; Figure 23B). Responses to leucine, KIC, alanine and arginine were significantly greater at 11.1 mmol/l compared with their effects at 5.6 mmol/l glucose (*p*<0.05 to *p*<0.001).

The insulin secretory responses of 1.4E7 and 1.2B4 cells to a range of agents were similar (Figure 24 and 25). Thus at 5.6 mmol/l glucose, both cell lines showed significant 1.2-1.7 fold insulin responses (*p*<0.05 to *p*<0.001) when tested with glyceraldehyde, arginine, tolbutamide or KCl. In the presence of 11.1 mmol/l glucose, the stimulatory actions of all agents tested was significantly increased (*p*< 0.05 to *p*<0.001; Figure 24B and 25B). Furthermore leucine, KIC and alanine each induced insulin secretion from 1.4E7 and 1.2B4 cells when tested at the higher glucose concentration (*p*<0.05 to *p*<0.01).

The effects of the K⁺-ATP channel opener, diazoxide on insulin release from 1.1B4, 1.1E7, 1,4E7 and 1.2B4 cells at 16.7 mmol/l glucose are show in Figure 26. Diazoxide (400µmol/l) caused a significant 20-50% inhibition of insulin release from cell lines (*p*<0.01 to *p*<0.001).

Inclusion of the voltage-dependent Ca²⁺ channel (VDCC) blocker, verapamil similarly inhibited insulin release by 20-40% (*p*<0.05 to *p*<0.001) from each of the cell lines (Figure 26). To evaluate the importance of extracellular Ca²⁺ for glucose-induced insulin release, a Ca²⁺-free buffer supplemented with the Ca²⁺-chelator, EGTA was employed. As shown in Figure 26 depletion of Ca²⁺ significantly decreased in insulin secretion by 20-40% (*p*<0.01 to *p*<0.001).

As shown in Figure 27, no significant differences were recorded between passage 17 and 40 in terms of basal insulin release and secretory responsiveness to glucose and KCl. Thus all four human islet- derived cell lines were able to maintain responsiveness (p<0.05 to p<0.001) to glucose or KCl at passage 40. As shown previously (Figure 9), cellular insulin content was stable at increasing passage number.

Insulin-secretory characterisation studies have clearly established that the four electrofusion-derived human islet cell lines express many of the important functional attributes of the parental B-cell. The following studies provide a fundamental molecular characterisation of the stimulus-secretion coupling pathways in these new cell lines.

Glucose transporter activity is generally considered as the first prerequisite for glucose-sensing in the pancreatic B-cell (Tiedge *et al*., 1993). Presence of glucose transporter (GLUT-1) was confirmed by Western blot analysis. Membrane preparations of parental cells (PANC-1 and Hup-T₃) and 1.1B4, 1.1E7, 1.4E7 and 1.2B4 cells were subjected to SDS-PAGE Western blotting analysis using a sensitive antibody against the GLUT-1 protein. As shown in Figure 28, the antibody detected a protein of approximately 48 kDa in each cell line. However, whereas 1.1B4, 1.1E7, 1.4E7 and 1.2B4 cells expressed significant levels of protein (particularly 1.1B4), the GLUT-1 transporter was not demonstrable in parental PANC-1 nor Hup-T₃ cells.

After glucose transport, the next step in the B-cell glucose-sensing mechanism involves the action of the glucose phosphorylating enzyme, glucokinase (Lenzen and Tiedge, 1994). Evaluation of glucokinase protein expression in parental cells and hybrids cells using Western blotting analysis is shown in Figure 29. By using a specific antibody directed against the glucokinase, a protein of 50 kDa was detected in each cell line. No glucokinase expression was demonstrable in parental PANC-1 and Hup-T₃ cells.

Further analysis of the glucose-sensing apparatus, attempted to link molecular and functional aspects of these novel insulin-secreting cell lines. The first step involved the characterisation of the glucose transport capacity of parental cells and each of the cell lines.

Consistent with the presence of functional membrane bound GLUT-1, 1.1B4, 1.1E7 and 1.4E7 cells exhibited different 3-O-methyl-D-[1-³H]glucose (3-O-MG) transport profiles at 1.1 and 16.7 mmol/l (Figure 30-32) compared with parental cells (PANC-1). As shown in Figure 30-32, the glucose uptake capacity was significantly higher (*p*<0.05 to *p*<0.001) at 16.7 mmol/l glucose compared with parental cells (PANC-1). When glucose uptake was tested at 1.1 mmol/l glucose, only 1.1B4 cells recorded a significantly higher (p<0.05) glucose uptake capacity compared with PANC-1 cells (Figure 30).

A broadly similar pattern of glucose uptake profiles was recorded in 1.2B4 cells (Figure 33). This cell line exhibited different 3-O-methyl-D-[1-³H]glucose (3-O-MG) transport profiles compared with parental cells (Hup-T₃). At 16.7 mmol/l, glucose uptake capacity was significantly higher in 1.2B4 cells compared with parental cells (Hup-T₃). However, when the cells were tested in 1.1 mmol/l 3-O-MG, no significant differences were recorded.

Glucose transport properties of each novel human islet cell line and parental cells were compared on the basis of their initial velocity of 3-O-MG uptake. As shown in Table 2, 1.2B4 cell line exhibited 6.5-fold increases in the initial velocity at 16.7 mmol/l glucose compared with 1.1 mmol/l glucose. Parental cells (Hup-T₃) exhibited a 5.2-fold increase in velocity. In the presence of 16.7 mmol/l glucose, 1.2B4 cells showed significantly (*p*<0.05) higher initial velocity uptake compared with Hup-T₃ cell lines. However, when 3-O-MG uptake was considered over 360 seconds, no significant differences were recorded in the half-maximal equilibration time either in 1.1 or 16.6 mmol/l 3-O-MG (Table 2).

The initial velocity of 3-O-MG uptake by 1.1B4, 1.1E7 and 1.4E7 cells was 5.1, 6.1 and 5.4-fold higher at 16.7 mmol/l than 1.1 mmol/l, respectively (Table 2). In comparison, PANC-1 cells exhibited 4.6-fold increase in the initial velocity at 16.7 mmol/l glucose. At both 1.1 mmol/l and 16.7 mmol/l, significant differences in 3-O-MG uptake were recorded between PANC-1 and 1.1B4 cells within the first 10 seconds. In 1.1E7 and 1.4E7 cells, significant differences (*p*<0.05) were only recorded at 16.7 mmol/l 3-O-MG. In terms of half-maximal equilibration time within 360 seconds of the measurements, only 1.1B4 showed a significant difference (*p*<0.05) (Table 3).

As shown in Figures 34 and 35, the parental immortal partners and hybrid human islet cell lines showed considerable differences in the relative contributions of glukokinase and hexokinase to the total glucose phosphorylating activity. Figure 34 shows the percentage of hexokinase activity represented (mean ± sem, n=3) 98.5 ± 9.3, 57.4 ± 4.6, 57.4 ± 14.5 and 72.6 ± 5.3 % of the total activity obtained from PANC-1, 1.1B4, 1.1E7 and 1.4E7 cells, respectively. In Hup-T₃ and 1.2B4 cells, the percentage of hexokinase activity represented (mean ± sem, n=6) 45.2 ± 5.5 and 63.8 ± 9.9 %, respectively (Figure 35). The percentage glucose phosphorylating activities of PANC-1, 1.1B4, 1.1E7 and 1.4E7 cells, attributed to glucokinase activity were 1.5 ± 0.8, 42.6 ± 9.6, 42.6 ± 11.3 and 27.4 ± 7.0 %, respectively. In Hup-T₃ cells and derived clonal 1.2B4 cells, the percentage glucokinase activities were 54.7 ± 7.4 and 36.1 ± 6.4 %, respectively. With the exception of 1.2B4 cells, all novel cell lines exhibited significantly higher (*p*<0.01-0.001) glucokinase and lower (*p*<0.01 to *p*<0.001)) hexokinase (expressed as percentage) compared with PANC-1 cells.

Experiments conducted to assess the amount of glucose being oxidised and utilised in the presence of varying concentrations of glucose and 5-thioglucose, showed interesting differences between the parental cells and the derived human islet cell lines.

Cell line 1.1B4 showed the highest levels of oxidation of glucose peaking at 95.4 ± 4.5 pmol/1000 cells/h at 11.1mmol/l glucose (Figure 36). At 16.7mmol/l levels of glucose oxidation closely resembled that at 5.6mmol/l. Inclusion of 5-thioglucose was able to inhibit the oxidation of glucose at both 1.1mmol/l and 16.7mmol/l glucose (Figure 37). The utilisation of glucose by this cell line followed a slightly different pattern with the highest utilisation levels being recorded at 136.5 ± 1.7 pmol/1000 cells/h at 16.7mmol/l glucose (Figure 38). 5-thioglucose did not affect utilisation at 1.1mmol/l glucose but was shown to reduce utilisation at 16.7mmol/l glucose (Figure 38)

Oxidation of glucose by cell line 1.1E7 was maximal at 16.7mmol/l glucose giving a value of 60.4 ± 5.4 pmol/1000 cells/h (Figure 36). Unlike 1.1E7 cells, 5-thioglucose only marginally inhibited glucose oxidation in 1.1B4 cells (Figure 37). However, the highest levels of glucose utilisation were recorded in this cell line, 181.9 ± 15.3 pmol/1000 cells/h at 16.7mmol/l glucose and again 5-thioglucose only affected utilisation at 16.7mmol/l (Figure 38).

Cell line 1.4E7 resembled 1.1B4, showing highest oxidation levels at 11.1mmol/l glucose with 5-thioglucose clearly inhibiting oxidation at both 1.1 and 16.7 mmol/l glucose (Figure 36 and 37). Utilisation of glucose by this cell line peaked at 16.7mmol/l glucose and was unaffected by inclusion of 5-thioglucose (Figure 38).

Cell line 1.2B4, cloned from parental Hup-T₃, showed poor oxidation of glucose at all concentrations tested, reaching only 7.0 ± 0.9 pmol/1000 cells/h at 16.7mmol/l (Figure 37). 5-thioglucose inhibited oxidation at 16.7mmol/l (Figure 40). Surprisingly, glucose utilisation rates for 1.2B4 closely resembled those of the other cell lines reaching a value of 127.5 ± 12.8 pmol/1000 cells/h at 16.7 mmol/l glucose (Figure 41). 5-thioglucose had no affect on the utilisation of glucose by this cell line.

Of the parental cell lines, glucose oxidation values for PANC-1 were far higher than for Hup-T₃. PANC-1 cells showed highest oxidation levels at 11.1mmol/l glucose with significant inhibition of oxidation at both 1.1 and 16.7mmol/l. As with the clonal progeny cell lines, PANC-1 glucose utilisation was highest at 16.7mmol/l - 163.7 ± 23.5 pmol/1000 cells/h. 5-thio-D-glucose was also able to inhibit utilisation only at this concentration. By contrast oxidation values for Hup-T₃ closely resembled that for it's progeny cell line 1.2B4, showing a maximum oxidation value of 12.6 ± 7.2 pmol/1000 cells/h at 11.1mmol/l glucose. In a separate experiment 5-thioglucose was able to inhibit oxidation at both 1.1 and 16.7mmol/l glucose. Again similar to 1.2B4, glucose utilisation values were similar to those of the other cells lines reaching a peak of 131.2 ± 13.2 pmol/1000 cells/h at 16.7mmol/l glucose. 5-thioglucose did not inhibit the utilisation of glucose in Hup-T₃ cells.

Table 3 shows the relative metabolism flux through oxidative glucose metabolism for the parental and each of the derived human islet cell lines. The significance of this index (ratio of glucose oxidation:glucose utilisation) is that glucose metabolism through oxidative pathway is most closely linked to insulin secretion (Malaisse, 1992). Ratios of all islet cell lines were significantly higher than parental Hup-T₃ or PANC-1 cells (Table 4). 1.1B4, 1.1E7 and 1.4E7 cells showed a significant increase in ratio on addition of 5-thioglucose.

Glucokinase protein was detected in all 6 cell lines studied and in contrast to insulin appeared as small punctate spots of fluorescence scattered around the cytoplasm (Figures 42). Antibody staining for this enzyme did appear to differ in intensity the between cell lines. Positive staining for insulin was detected at high intensity levels in cell lines 1.1B4, 1.4E7 with lesser intensity in 1.1E7 and 1.2B4 (Figures 43). From the images it can be seen that insulin protein is located at various site throughout the cytoplasm, appearing under fluorescence as diffuse bright patches. In the parental cell lines (PANC-1 and Hup-T₃), no cells out of the entire field showed any staining for insulin. Similar to insulin staining, islet amyloid polypeptide (IAPP) appeared as bright spots located to the cytoplasm. Again all four cell lines showed a positive signal for IAPP with higher levels of staining in 1.1B4, 1,1E7 and 1.4E7 (Figures 44). No positive staining was seen above background for either glucagon or somatostatin in any of the cell lines tested (Figures 45 and 46). Immunostaining characteristics of all 6 cell lines is summarised in Table 5.

Each described embodiment describes the production and characterization of hybrid human insulin-secreting cell lines generated by the electrofusion technique. Fusions were carried out using PANC-1 or Hup-T₃ immortal partner cells resulting in production of 38 hybrids (Figure 1). Of the 27 insulin-secreting clones produced, the first four have been characterised through a series of morphological and secretory studies, analyzing the ability of each novel cell line to respond to glucose and other regulators of B-cell function. The four novel cell lines generated by electrofusion, and characterised in this Chapter are provisionally designated as 1.1B4, 1.1E7, 1.4E7 and 1.2B4 cells. Attempts to generate hybrids using TRM-1, HAP-5 or B6 as immortal partner cells were thwarted by failure of these cells to continue to proliferate in tissue culture.

The three cell lines generated from PANC-1 (1.1B4, 1.1E7 and 1.4E7) and the cell line produced by electrofusion with Hup-T₃ cells (1.2B4) all grew as monolayers in tissue culture showing a pavemental pattern when confluent, characteristic of epithelial cells. The respective doubling times were approximately 23 ± 1.7 hours for 1.1B4 cells, 23 ± 1.9 for 1.1E7 cells, 26 ± 1.5 hours for 1.4E7 cells and 36 ± 2.1 hours for 1.2B4 cells (n=4). These inherent differences in the growth pattern support the view that each represent a unique and novel clonal cell line. The four cell lines were shown to express insulin, glucokinase and IAPP by immunocytochemistry. No visible staining was evident for glucagon or somatostatin

Glucose is a principal regulator of insulin secretion from the pancreatic B-cell (Meglasson and Matschinsky, 1986). Static incubation of the various novel human islet cell lines over 60 min with a range of increasing glucose concentrations revealed substantial differences in insulin secretory responsiveness of each cell line. 1.1B4 cells showed a more appropriate stepwise pattern of insulin output compared with other cell lines, with a threshold at 5.6 mmol/l glucose. 1E7 and 1.4E7 cell lines showed peak responses at 11.1 and 5.6 mmol/l glucose, respectively. The falling off of insulin output at higher glucose concentration possibly reflects the dual action of glucose of increasing intracellular Ca²⁺ sequestration and Ca²⁺ influx, as has been implicated for the initial transient inhibitory phase demonstrable in normal B-cells (Hellman *et al*., 1992). This phenomenon is also observed in other animal-derived insulin-secreting cell lines including HIT-T15, BRIN-BG5 and BRIN-BG7 (Poitout *et al*., 1996; McClenaghan *et al*., 1996b). In contrast, to the other three electrofusion derived human islet cell lines, no significant insulin secretory response to glucose was observed in 1.2B4 cells under standard incubation conditions.

The insulin output in response to glucose was significantly enhanced in the presence of 200 µmol/l IBMX in 1.1B4, 1.1E7 and 1.4E7 cells. The phosphodiesterase inhibitor IBMX enhanced insulin secretion by 20 to 60% and shifted the threshold to 5.6 mmol/l glucose in 1.1E7 cells. The action mediated by IBMX indicates that the cells utilize IBMX to elevate cAMP and potentiate Ca²⁺-mediated insulin release as in normal pancreatic B-cells (Hellman *et al*., 1992). IBMX also modestly enhanced insulin secretion from 1.1B4 and 1.4E7 cells in the absence of glucose. This stimulatory effect most probably reflects the ability of higher concentrations of cyclic AMP to stimulate secretion through mobilization of intracellular Ca²⁺ and promotion of Ca²⁺ influx, as also noted for the normal pancreatic B-cell (Hellman, *et al*., 1992). Hup-T₃-derived 1.2B4 cells were insensitive to IBMX.

Inclusion of 2 mmol/l 5-thioglucose, a glucose analog that is a potent inhibitor of hexokinase, caused a shift in glucose-insulin dose response for each cell line. In 1.1B4, 1.1E7 and 1.2B4 cells, the effects were significant (*p*<0.01 to *p*<0.001), consisting of a 20 to 50% reduction of insulin secretion at 0 mmol/l to 11.1 mmol/l glucose. In addition, inclusion of 5-thioglucose caused a.shift in glucose responsiveness of 1.1E7 cells with a threshold concentration for stimulation of insulin release at 5.6 mmol/l glucose. In the presence of 5-thioglucose, glucose-stimulated insulin release also became apparent in Hup-T₃-derived 1.2B4 cells. These observations suggest that the beneficial effects of 5-thioglucose on secretion are due to inhibition of hexokinase with a greater proportion of glucose flux catalyzed by glucokinase such that the signaling potency of glucose metabolism is increased (Hohmeier *et al*., 1997). Insulin output and glucose responsiveness of 1.4E7 cells was not affected by 5-thioglucose.

Further characterisation of each cell line involved evaluation of the insulinotropic activity of a number of amino acids including electrically neutral amino acids (L-alanine, L-leucine) and cationic amino acids (L-arginine). Although many studies have been performed to elucidate the mechanism of amino acid-induced insulin secretion (Henquin and Meissner, 1981; Malaisse *et al*., 1991), the effects of many amino acids including L-alanine and glycine are poorly understood. When tested at a non-stimulatory (5.6 mmol/l) glucose concentration, L-arginine significantly stimulated insulin secretion in each of human islet-derived cell lines. Significant effects of L-leucine and L-alanine on insulin secretion at 5.6 mmol/l was observed only in 1.1B4 cells possibily reflecting that these amino acids serve as potentiators rather than powerful initiators of insulin release on human islet cells (Yada, 1994).

When tested at 11.1 mmol/l glucose, each amino acid tested, leucine, KIC, arginine and alanine caused significant increases in insulin release. This indicates that the novel cells possess each of the various amino acid transport systems and related signal recognition pathways described in normal pancreatic B-cells (Hellman *et al*., 1971; Yada, 1994). Additionally these findings demonstrate that the amino acids tested can utilise stimulatory glucose for their insulinotropic actions on these novel human islet cells. This confirms recent observations that amino acids may act in a glucose-dependent manner, namely that glucose acts as a fuel through its metabolism, which may potentiate the uptake and utilization of certain amino acids (McClenaghan and Flatt, 1999). It is also interesting to note that glyceraldehyde, a triose sugar, was able to significantly stimulate insulin secretion from each of the novel cell lines. Like glucose, glyceraldehyde stimulates insulin release by its glycolytic metabolism and subsequent generation of ATP (Halban and Wollheim, 1980). Depolarisation of plasma membrane by a high concentration of KCl or blockade of ATP-sensitive potassium (K⁺-ATP) channel with glibenclamide or tolbutamide also provoked insulin release from the novel cell lines as previously established from normal B-cell (Kramer *et al*., 1996).

In order to study further the role of glycolysis and K⁺-ATP channels in glucose-induced insulin secretion, effects of diazoxide were evaluated. This agent is known to open K⁺-ATP channels and thus acts to repolarize the B-cell membrane and inhibit insulin release (Henquin *et al*., 1992). As expected, this agent significantly reduced insulin output of all four cell lines at 16.7 mmol/l glucose, indicating the importance of the K⁺-ATP channel in regulation of insulin secretion from each of the clonal B-cell lines.

Ca²⁺ has been known for many years to play a important role in nutrient-induced insulin secretion (Hellman, 1975). The importance of Ca²⁺-influx in glucose-induced insulin secretion was highlighted through incubations performed in the absence of extracellular Ca²⁺ and with the Ca²⁺ chelator EGTA. Depletion of Ca²⁺ significantly inhibited insulin secretion at 16.7 mmol/l glucose from each of the four cell lines. Blockage of voltage-dependent Ca²⁺ channels (VDCC) using verapamil similarly reduced glucose-induced insulin secretion. These results clearly indicate the importance of Ca²⁺ and voltage-dependent Ca²⁺ channels in the regulation of insulin secretion from the novel hybrid cell lines. It is therefore possible to infer from these functional studies that the cells express K⁺-ATP channels, SUR, Kir and VDCC.

Equally importantly, insulin content and secretory responses of all four human islet B-cell lines were shown to remain stable at low and high passage number. Thus cellular insulin content, basal insulin release and secretory effects of glucose and KCl were similar at passages 17 and 40. Such functional stability appears to be a significant attribute of electrofusion-derived compared with transfected pancreatic B-cell lines.

Availability of molecular probes enabled Western blotting analysis of all four novel human islet cell lines and parental cell lines using a specific antibodies directed against the GLUT-1 transporter protein and against glucokinase. Proteins of approximately 48 kDa and 50 kDa, respectively were detected in all four cell lines but not in parental PANC-1 nor Hup-T₃ cells. The GLUT-1 transporter is known to play a important role in glucose uptake, acting in conjunction with glucokinase to form the so called B-cell glucose-sensing mechanism in pancreatic B-cells (Shibasaki *et al*., 1990).

Measurements of GLUT-1 and glucokinase protein were accompanied by functional measures of activity assessed by evaluation of glucose transport and contribution of glucokinase/hexokinase to the total glucose phosphorylating activities of the various cell lines. These studies revealed that the novel human islet clones exhibited efficient glucose transport characteristics which, unlike parental cells, were not overwhelmed by increasing the extracellular glucose concentration from 1:1 to 16.7 mmol/l. This characteristic has previously been defined as a distinguishing feature of the pancreatic B-cell (Hellman *et al*., 1974). More recent molecular studies consider glucose transport not to be rate limiting in normal pancreatic B-cells except under most extreme circumstances (Ohneda *et al*., 1994)).

Glucokinase is generally believed to be one of the most important determinants of glucose sensitivity in the pancreatic B-cell (Lenzen and Tuedge,1994). In particular, a high contribution of glucokinase to total phosphorylating activity (as apposed to hexokinase) is a landmark feature of normally functioning insulin-secreting cells. In the present study, glucokinase contributed < 2% to the glucose phosphorylating activity of parental PANC-1 cells. Consistent with Western blotting data, the glucokinase contribution of derived 1.1B4, 1.1E7, 1.4E7 ranged between 27-43%. The other cell line, 1.2B4 was characterised by 36% contribution of glucokinase to total phosphorylating activity. There measures compare quite favorably to the value of 50% reported to the normal B-cells (Lenzen, 1990), and clearly indicate efficient transport and metabolism of glucose in the human partner B-cells.

Metabolism of glucose by the B-cell represents a culmination of glucose transport and phosphorylation activities. Evaluation of the metabolic response in parental and derived cell lines indicated a stepwise increase in glucose oxidation/utilisation with increasing glucose concentration. Inhibition of hexokinase activity using 5-thioglucose also showed inhibitory effects as reported elsewhere (Hohmeier *et al*., 1997). Since glucose stimulation of insulin secretion is tightly linked to ATP generation, relative flux of glucose metabolism through oxidative pathways has been considered as a particularly noteworthy indicator of the functional integrity of the pancreatic B-cell (Malaisse, 1992). Calculation of the ratios glucose oxidation:glucose utilisation revealed higher values in each of the hybrid cell compared with parental PANC-1 or Hup-T₃ cells. Such effects were broadly linked to relative glucokinase activities of the various cell lines.

Although the immunohistochemistry results were only part of early studies on the expression of B-cell markers, it is reassuring that positive staining for insulin was detected by the insulin antiserum in all of the clonal hybrid cell lines. As seen from the FITC images cells do not appear to have uniform insulin staining. This probably reflects the inability of this particular approach to detect low levels of insulin present in some of the cells. Staining for insulin using the same antibody in rat pancreas showed strong fluorescent signal (results not shown) but monolayers of B-cells contained far lower levels of insulin, estimated less than 5% of normal pancreatic islet cells.

Glucokinase protein was detected by immunocytochemistry in all cell lines as small, granular spots of flourescence scattered around the cytoplasm. As with insulin it was difficult to visually quantify the levels of glucokinase from these experiments, however a careful examination of each cell line indicated that levels of positive staining were again augmented in hybrid islet cells compared with the parental cell lines. IAPP was found in all cell lines and also appeared as small spots of positive staining around the cytoplasm and was less intense than the staining seen for glucokinase. No positive staining for either glucagon or somatostatin was seen in any of the cell lines tested supporting the view that the hybrids represent a pure population of clonal pancreatic B-cells.

The present invention reports the generation and characterisation of four novel electrofusion-derived human insulin-secreting pancreatic B-cell lines. Functional assessment of these unique cells indicates that many of the features of the normal human pancreatic B-cell have been inherited by the hybrid cells from the human donors (Table 6).

DNA identity profiling of the four electrofusion derived human insulin-secreting pancreatic B cell lines indicates that cell lines 1.1E7, 1.4E7 and 1.1B4 have similar DNA profiles which 1.2B4 has a different profile.

Tables 7,8,9 and 10 show the identity profile results of 1.1E7, 1.4E7, 1.1B4 and 1.2B4 respectively.

These profiles indicate that cell lines produced by the electrofusion process which show features of normal pancreatic B cells do not require to share identical genetic identity.

The cell lines produced appear to have superior functional qualities to many existing animal cell lines which can be attributed to presence of key components of the normal pancreatic B-cell stimulus-secretion coupling pathway. These various observations, combined with functional stability, indicate that electrofusion-derived human pancreatic B-cell clones will be of substantial benefit for diabetes research. In addition, these cell lines will enable the use of cellular engineering using electrofusion-derived pancreatic B-cells to provide therapy for type 1 diabetes.

## Claims

1. A human pancreatic cell line produced by electrofusion of normal human islet cells with cells from at least one immortal human cell line wherein the human pancreatic cell-line is capable of secreting insulin.

2. A human pancreatic cell line capable of secreting insulin chosen from the group of cell lines consisting of the cell-line deposited under Accession No 00112811 at the European Collection of Cell Cultures (ECACC), CAMR, Salisbury, Wiltshire on 28 November 2000 and the cell-lines deposited under Accession Nos PTA 3523, PTA 3524 and PTA 3525 at the American Type Culture Collection, 10801 University Boulevard, Manassas, Virginia 20100-2209, USA on 17 July 2001.

3. A process for the production of human pancreatic cell lines capable of secreting insulin, the process including the steps of electrofusing a mixture of normal human islet cells with cells from at least one immortal human cell line and incubating the mixture to generate hybrid cells.

4. The process as claimed in claim 3 wherein the human islet cells and immortal human cells are mixed in a 1:1 ratio.

5. The process as claimed in claims 3 or 4 wherein electrofusion occurs in a helical chamber.

6. The process as claimed in any of claims 3 to 5 wherein the electrofusion step includes exposing the cells to a first pulse phase of AC field, a second pulse phase of DC field and a third pulse phase of AC field.

7. The process as claimed in claim 6 wherein the first pulse phase is comprised of 7V, 2MHZ of AC field for 30 seconds.

8. The process as claimed in claims 6 or 7 wherein the second pulse phase is comprised of 60V, triple pulses of DC field with each of the triple pulses being 15 seconds in duration.

9. The process as claimed in claim 6, 7 or 8 wherein the third pulse phase is comprised of 7V, 2MHZ of AC field for 30 seconds.

10. The process as claimed in any of claims 3 to 9 wherein the media in which the cells are incubated comprises hypoxanthine, aminopterin and thymidine.

11. The process as claimed in claim 10 wherein hypoxanthine is present in the incubating media at a concentration of between 0.05µmol/l to 0.5µmol/l.

12. The process as claimed in claim 10 or 11 wherein aminopterin is present in the incubating media at a concentration of between 0.2-0.6 µmol/l.

13. The process as claimed in claims 10, 11 or 12 wherein thymidine is present in the incubating media at a concentration of between 10-20 µmol/l.

14. The process as claimed in any of claims 3 to 13 wherein incubation is carried out in the presence of at least one secretagogue chosen from the group comprising glucose, glyceraldehyde, arginine, leucine and alanine.

15. The process as claimed in any of claims 3 to 14 wherein incubation is carried out in the presence of at least one substance chosen from the group comprising KCl, IBMX, thioglucose, tolbutamide, diazoxide and verapamil.

16. A cell line produced by a process as claimed in any of claims 3 to 15 which exhibits glucose transport characteristics as efficient as normal pancreatic B cells.

17. A cell line produced by a process claimed in any of claims 3 to 15 which exhibits glucose phosphorylating activity consistent with normal pancreatic B cells.

18. Insulin producing cells produced by the process as claimed in any of claims 3 to 15 for use in gene therapy for type 1 diabetes.

19. The use of insulin producing cells produced by the process as claimed in any of claims 3 to 15 in the preparation of a medicament for the treatment of diabetes.

20. Use of a cell-line as claimed in any of claims 1, 2, 16 or 17 in the preparation of a medicament for the treatment of diabetes.

21. Use of a cell-line as claimed in any of claims 1, 2, 16 or 17 for the production of insulin.

## Patentansprüche

1. Eine menschliche Pankreaszell-Linie, die durch Elektrofusion normaler menschlicher Inselzellen mit Zellen von mindestens einer unsterblichen menschlichen Zelt-Linie erzeugt wird, wobei die menschliche Pankreaszell-Linie Insulin absondern kann.

2. Menschliche Pankreaszell-Linie, die Insulin absondern kann, die aus der Gruppe von Zell-Linien ausgewählt ist, welche aus der Zell-Linie, die unter der Zugangsnr. 00112811 bei der Europäischen Sammlung von Zellkulturen (ECACC), CAMR, Salisbury, Wiltshire am 28. November 2000 hinterlegt wurde, und den Zell-Linien, die unter den Zugangsnr. PTA 3523, PTA 3524 und PTA 3525 bei der American Type Culture Collection, 10801 University Boulevard, Manassas, Virginia 20100-2209, USA am 17. Juli 2001 hinterlegt wurden, besteht.

3. Ein Verfahren zur Erzeugung von menschlichen Pankreaszell-Linien, die Insulin absondern können, wobei das Verfahren die Schritte des Elektrofusionierens einer Mischung normaler menschlicher Inselzellen mit Zellen von mindestens einer unsterblichen menschlichen Zell-Linie und des Inkubierens der Mischung, um Hybridzellen herzustellen, umfasst.

4. Verfahren gemäß Anspruch 3, wobei die menschlichen Inselzellen und die unsterblichen menschlichen Zellen in einem Verhältnis von 1:1 gemischt werden.

5. Verfahren gemäß Anspruch 3 oder 4, wobei die Elektrofusion in einer Helixkammer geschieht.

6. Verfahren gemäß einem der Ansprüche 3 bis 5, wobei der Schritt des Elektrofusionierens das Exponieren der Zellen gegenüber einer ersten Pulsphase eines Wechselstromfelds, einer zweiten Pulsphase eines Direktstromfelds und einer dritten Pulsphase eines Wechselstromfelds umfasst.

7. Verfahren gemäß Anspruch 6, wobei die erste Pulsphase 7 V, 2 MHz eines Wechselstromfelds für 30 Sekunden beinhaltet.

8. Verfahren gemäß Anspruch 6 oder 7, wobei die zweite Pulsphase 60 V, Dreifachpulse eines Direktstromfelds beinhaltet, wobei jeder der Dreifachpulse 15 Sekunden dauert.

9. Verfahren gemäß Anspruch 6, 7 oder 8, wobei die dritte Pulsphase 7 V, 2 MHz eines Wechselstromfelds für 30 Sekunden beinhaltet.

10. Verfahren gemäß einem der Ansprüche 3 bis 9, wobei die Medien, in denen die Zellen inkubiert werden, Hypoxanthin, Aminopterin und Thymidin beinhalten.

11. Verfahren gemäß Anspruch 10, wobei Hypoxanthin in den Inkubationsmedien in einer Konzentration von zwischen 0,05 µmol/l und 0,5 µmol/l vorhanden ist.

12. Verfahren gemäß Anspruch 10 oder 11, wobei Aminopterin in den Inkubationsmedien in einer Konzentration von zwischen 0,2 µmol/l und 0,6 µmol/l vorhanden ist.

13. Verfahren gemäß Anspruch 10, 11 oder 12, wobei Thymidin in den Inkubationsmedien in einer Konzentration von zwischen 10 µmol/l und 20 µmol/l vorhanden ist.

14. Verfahren gemäß einem der Ansprüche 3 bis 13, wobei die Inkubation in der Gegenwart von mindestens einem Sekretagogum, das aus der Gruppe ausgewählt ist, die Glucose, Glycerinaldehyd, Arginin, Leucin und Alanin beinhaltet, durchgeführt wird.

15. Verfahren gemäß einem der Ansprüche 3 bis 14, wobei die Inkubation in der Gegenwart von mindestens einer Substanz, die aus der Gruppe ausgewählt ist, die KCI, IBMX, Thioglucose, Tolbutamid, Diazoxid und Verapamil beinhaltet, durchgeführt wird.

16. Eine Zell-Linie, die durch ein Verfahren gemäß einem der Ansprüche 3 bis 15 erzeugt wird, die Glucosetransportcharakteristiken, die so wirksam wie normale pankreatische B-Zellen sind, vorzeigt.

17. Zell-Linie, die durch ein Verfahren gemäß einem der Ansprüche 3 bis 15 erzeugt wird, die eine Glucosephosphorylierungsaktivität, die normalen pankreatischen B-Zellen entspricht, vorzeigt.

18. Insulin erzeugende Zellen, die durch das Verfahren gemäß einem der Ansprüche 3 bis 15 erzeugt werden, zur Verwendung in der Gentherapie für Typ-1-Diabetes.

19. Die Verwendung von Insulin erzeugenden Zellen, die durch das Verfahren gemäß einem der Ansprüche 3 bis 15 erzeugt wurden, bei der Zubereitung eines Medikaments zur Behandlung von Diabetes.

20. Die Verwendung einer Zell-Linie gemäß einem der Ansprüche 1, 2, 16 oder 17 bei der Zubereitung eines Medikaments zur Behandlung von Diabetes.

21. Die Verwendung einer Zell-Linie gemäß einem der Ansprüche 1, 2, 16 oder 17 für die Erzeugung von Insulin.

## Revendications

1. Une lignée cellulaire pancréatique humaine produite par électrofusion de cellules d'îlots humains normales avec des cellules provenant d'au moins une lignée cellulaire humaine immortelle dans laquelle la lignée cellulaire pancréatique humaine est capable de sécréter de l'insuline.

2. Une lignée cellulaire pancréatique humaine capable de sécréter de l'insuline sélectionnée dans le groupe de lignées cellulaires consistant en la lignée cellulaire déposée sous le N° d'Accès 00112811 à the European Collection of Cell Cultures (ECACC), CAMR, Salisbury, Wiltshire le 28 novembre 2000 et les lignées cellulaires déposées sous les N^{os} d'Accès PTA 3523, PTA 3524 et PTA 3525 à the American Type Culture Collection, 10801 University Boulevard, Manassas, Virginia 20100-2209, Etats-Unis le 17 juillet 2001.

3. Un procédé destiné à la production de lignées cellulaires pancréatiques humaines capables de sécréter de l'insuline, le procédé comprenant les étapes d'électrofusion d'un mélange de cellules d'îlots humains normales avec des cellules provenant d'au moins une lignée cellulaire humaine immortelle et d'incubation du mélange pour générer des cellules hybrides.

4. Le procédé tel que revendiqué dans la revendication 3 dans lequel les cellules d'îlots humains et les cellules humaines immortelles sont mélangées dans un rapport 1/1.

5. Le procédé tel que revendiqué dans les revendications 3 ou 4 dans lequel l'électrofusion a lieu dans une chambre hélicoïdale.

6. Le procédé tel que revendiqué dans n'importe lesquelles des revendications 3 à 5 dans lequel l'étape d'électrofusion comprend l'exposition des cellules à une première phase d'impulsions de champ CA, à une deuxième phase d'impulsions de champ CC et à une troisième phase d'impulsions de champ CA.

7. Le procédé tel que revendiqué dans la revendication 6 dans lequel la première phase d'impulsions se compose de 7 V, 2 MHZ de champ CA pendant 30 secondes.

8. Le procédé tel que revendiqué dans les revendications 6 ou 7 dans lequel la deuxième phase d'impulsions se compose de 60 V, d'impulsions triples de champ CC, chacune des impulsions triples étant d'une durée de 15 secondes.

9. Le procédé tel que revendiqué dans la revendication 6, la revendication 7 ou la revendication 8 dans lequel la troisième phase d'impulsions se compose de 7 V, 2 MHZ de champ CA pendant 30 secondes.

10. Le procédé tel que revendiqué dans n'importe lesquelles des revendications 3 à 9 dans lequel les milieux dans lesquels les cellules sont incubées comportent de l'hypoxanthine, de l'aminoptérine et de la thymidine.

11. Le procédé tel que revendiqué dans la revendication 10 dans lequel l'hypoxanthine est présente dans les milieux d'incubation à une concentration d'entre 0,05 µmol/l et 0,5 µmol/l.

12. Le procédé tel que revendiqué dans la revendication 10 ou la revendication 11 dans lequel l'aminoptérine est présente dans les milieux d'incubation à une concentration d'entre 0,2 et 0,6 µmol/l.

13. Le procédé tel que revendiqué dans les revendications 10, 11 ou 12 dans lequel la thymidine est présente dans les milieux d'incubation à une concentration d'entre 10 et 20 µmol/l.

14. Le procédé tel que revendiqué dans n'importe lesquelles des revendications 3 à 13 dans lequel l'incubation est effectuée en présence d'au moins un sécrétagogue sélectionné dans le groupe comportant glucose, glycéraldéhyde, arginine, leucine et alanine.

15. Le procédé tel que revendiqué dans n'importe lesquelles des revendications 3 à 14 dans lequel l'incubation est effectuée en présence d'au moins une substance sélectionnée dans le groupe comportant KCI, IBMX, thioglucose, tolbutamide, diazoxide et vérapamil.

16. Une lignée cellulaire produite par un procédé tel que revendiqué dans n'importe lesquelles des revendications 3 à 15, laquelle présente des caractéristiques de transport de glucose aussi efficaces que les cellules B pancréatiques normales.

17. Une lignée cellulaire produite par un procédé revendiqué dans n'importe lesquelles des revendications 3 à 15, laquelle présente une activité de phosphorylation de glucose consistante avec les cellules B pancréatiques normales.

18. Des cellules productrices d'insuline produites par le procédé tel que revendiqué dans n'importe lesquelles des revendications 3 à 15 destinées à être utilisées dans la thérapie génique pour le diabète de type 1.

19. L'utilisation de cellules productrices d'insuline produites par le procédé tel que revendiqué dans n'importe lesquelles des revendications 3 à 15 dans la préparation d'un médicament pour le traitement du diabète.

20. Utilisation d'une lignée cellulaire telle que revendiquée dans n'importe lesquelles des revendications 1, 2, 16 ou 17 dans la préparation d'un médicament pour le traitement du diabète.

21. Utilisation d'une lignée cellulaire telle que revendiquée dans n'importe lesquelles des revendications 1, 2, 16 ou 17 pour la production d'insuline.
